# EUROPEAN PATENT APPLICATION

(11) **EP 3 597 691 A1**
(43) Date of publication of application: **22.01.2020**
(21) Application number: 19782462.6
(22) Date of filing: 22.04.2019
(51) Int. Cl.: C08J 3/00, A61K 8/06, A61K 8/42, A61K 8/81, A61Q 1/02, A61Q 5/12, A61Q 17/04, A61Q 19/00, A61Q 19/02

(54) **NOVEL COMPOSITE AND EMULSIFIED COMPOSITION**

(30) Priority: 27.04.2018 JP 2018086864; 30.11.2018 JP 2018225690
(71) Applicant: Kokyu Alcohol Kogyo Co., Ltd., Narita-shi, Chiba 287-0225 (JP)
(72) Inventor: The designation of the inventor has not yet been filed
(74) Representative: Cabinet Nuss
(86) International application number: PCT/JP2019/017030
(87) International publication number: WO 2019/208497

(57) **Abstract**

An object of the invention is to provide a novel complex or an emulsified composition that can be used as an emulsifier. A complex formed by bonding an amide alcohol and a carboxy group-containing polymer exerts an excellent water-in-oil type or oil-in-water type emulsifying power.

## Description

### [Technical Field]

The present invention relates to a novel complex having emulsifying properties, and to a water-in-oil emulsified composition, and an oil-in-water emulsified composition.

### [Background Art]

As an emulsified composition for cosmetic materials, conventionally, cream, milky lotion, beauty essence, etc. in which various oily materials and aqueous materials are emulsified are widely used. In producing such an emulsified composition, in addition to processing materials under strictly set various conditions, it has been necessary to use a surfactant in order to ensure temporal stability of the emulsified composition. In recent years, however, even higher safety is expected for cosmetic materials, and from this viewpoint, presence of a surfactant may be a problem.

To solve such a problem, as a water-in-oil emulsified composition without using a surfactant, a water-in-oil emulsified composition which is characterized by comprising, for example, one or more kinds of oils selected from the group consisting of fatty acids and higher alcohols, one or more kinds of polymers soluble to said oils, and one or more kinds of compounds selected from the group consisting of inorganic salts, organic acid salts, amino acids and salts thereof, and not comprising a surfactant, has been proposed (Patent Document 1). However, PVP (polyvinylpyrrolidone) used as one of more kinds of oil-soluble polymers tends to exhibit stickiness, and there is a problem in feeling of use.

An emulsifier capable of providing a water-in-oil emulsion without using a surfactant, and having good feeling of use, has not yet been obtained.

Incidentally, a secondary amide including an amide alcohol has been studied as an agent imparting moisture resistance, and in a composition using this, formulation wherein a secondary amide is used together with a hydrophilic acrylic polymer (a carboxy group-containing polymer) as a thickener has been prepared (Patent Document 2). However, the secondary amide used in the emulsion prepared in this document does not contain an amide alcohol, and in the preparation method described in this document, no complex is formed, and the use of a common emulsifier (surfactant) is required in order to prepare an emulsified composition.

### [Citation List]

### [Patent Document]

[Patent Document 1] JP A 2003-252723
[Patent Document 2] JP A H1-502116

### [Disclosure of Invention]

### [Problems to Be Solved by the Invention]

In view of the above-described problems in the prior art, an object of the present invention is to provide an agent and a complex exhibiting a water-in-oil type or oil-in-water type emulsifying power, without using a general emulsifier (surfactant). It is a further object of the present invention to provide a water-in-oil or oil-in-water emulsion utilizing such a water-in-oil type or oil-in-water type emulsifying power. Another object of the present invention is to provide an emulsifier capable of obtaining a water-in-oil or oil-in-water emulsion excellent in stability, and a stable water-in-oil or oil-in-water emulsion. Moreover, a further object of the present invention is to provide an emulsifier capable of obtaining a water-in-oil or oil-in-water emulsion excellent in feeling of use on the skin and hair, and a water-in-oil or oil-in-water emulsion having excellent feeling of use.

### [Means for Solving the Problems]

During extensive research to solve the above problems, the present inventors have discovered that emulsification is possible by using an amide alcohol and a polymer containing a carboxy group, and that emulsifying properties are enhanced by using a polyhydric alcohol; and as a result of further research, the inventors have completed the present invention.

That is, the present invention relates to (1) to (12) below.
(1) An emulsified composition, comprising an amide alcohol represented by formula (I): 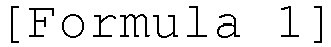 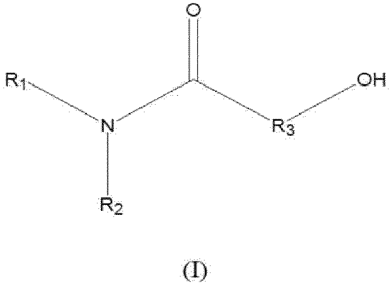 wherein
   R₁ is a C6-C22 hydrocarbon group,
   R₂ is H, or a C6-C22 hydrocarbon group,
   R₃ is a linear or branched C2-C21 hydrocarbon group,
   and a carboxy group-containing polymer.
(2) The emulsified composition according to (1), further comprising one or more polyhydric alcohols.
(3) The emulsified composition according to (2), wherein the polyhydric alcohol is pentylene glycol and/or 2-ethyl-1,3-hexanediol.
(4) The emulsified composition according to any one of (1) to (3), further comprising a higher fatty acid.
(5) The emulsified composition according to any one of (1) to (4), further comprising an oil gelling agent.
(6) The emulsified composition according to any one of (1) to (5), which is of W/O type.
(7) The emulsified composition according to any one of (1) to (6), which is of O/W type.
(8) A complex formed by bonding an amide alcohol represented by formula (I): 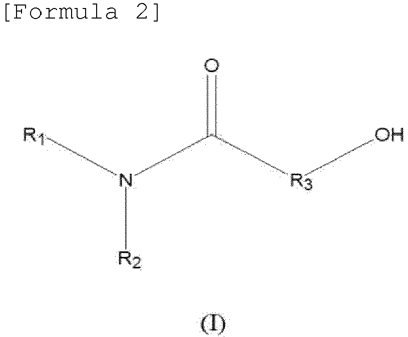 wherein
   R₁ is a C6-C22 hydrocarbon group,
   R₂ is H, or a C6-C22 hydrocarbon group,
   R₃ is a linear or branched C2-C21 hydrocarbon group,
   and a carboxy group-containing polymer,
   and a higher fatty acid.
(9) The complex according to (8), further comprising one or more polyhydric alcohols.
(10) A method for producing an emulsified composition according to Claim 1, wherein an oil phase comprising an amide alcohol represented by formula (I): 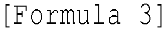 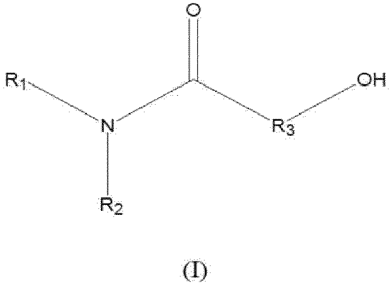 wherein
   R₁ is a C6-C22 hydrocarbon group,
   R₂ is H or a C6-C22 hydrocarbon group,
   R₃ is a linear or branched C2-C21 hydrocarbon group,
   and an aqueous phase comprising a carboxy-group containing polymer are mixed.
(11) The method for producing an emulsified composition according to (10), wherein the emulsified composition is of W/O type.
(12) The method for producing an emulsified composition according to (11), wherein the emulsified composition is of O/W type.

That is, the present invention relates to [1] to [21] below.
[1] A method for producing a W/O emulsion, wherein an oil phase comprising an amide alcohol represented by formula (I): 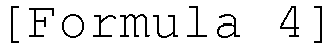 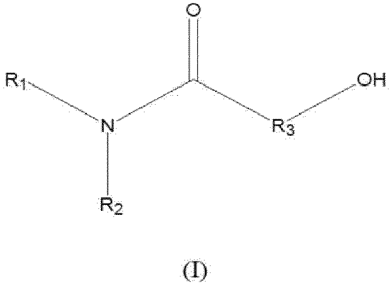 wherein
   R₁ is a C6-C22 hydrocarbon group,
   R₂ is H, or a C6-C22 hydrocarbon group,
   R₃ is a linear or branched C2-C21 hydrocarbon group,
   and an aqueous phase comprising a carboxy group-containing polymer are mixed.
[2] The method according to [1], which comprises neutralizing by adding a neutralizing agent.
[3] The method according to [2], wherein the aqueous phase is neutralized by adding a neutralizing agent, and the aqueous phase and the oil phase are mixed.
[4] The method according to [2] wherein, after mixing the aqueous phase and the oil phase, the mixture is neutralized by adding a neutralizing agent.
[5] The method according to any one of [1] to [4], wherein the oil phase further comprises a higher fatty acid.
[6] The method according to any one of [1] to [5], wherein the carboxy group-containing polymer has a molecular weight of 500,000 to 3,000,000 and a carboxy group content of 50 to 70%.
[7] The method according to any one of [1] to [6], wherein the carboxy group-containing polymer is a carboxyvinyl polymer and/or an alkyl-modified carboxyvinyl polymer.
[8] The method according to any one of [1] to [7], wherein the oil phase further comprises a higher fatty acid.
[9] A W/O emulsified composition, comprising an amide alcohol represented by formula (I): 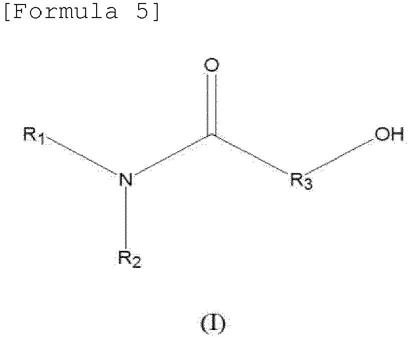 wherein
   R₁ is a C6-C22 hydrocarbon group,
   R₂ is H, or a C6-C22 hydrocarbon group,
   R₃ is a linear or branched C2-C21 hydrocarbon group,
   and a carboxy group-containing polymer.
[10] The W/O emulsified composition according to [9], which comprises no surfactant or comprises less than 2.0 mass% of a surfactant.
[11] The W/O emulsified composition according to [9] or [10], further comprising a higher fatty acid.
[12] The W/O emulsified composition according to any one of [9] to [11], further comprising an oil gelling agent.
[13] The W/O emulsified composition according to [12], wherein the oil gelling agent is an organically modified clay mineral and/or a polymer ester of sugar and fatty acid.
[14] The W/O emulsified composition according to any one of [9] to [13], further comprising pentylene glycol.
[15] The W/O emulsified composition according to any one of [9] to [14], wherein the weight ratio of nonpolar oil/(nonpolar oil + polar oil) is greater than 0.5.
[16] A complex formed by bonding an amide alcohol represented by formula (I): 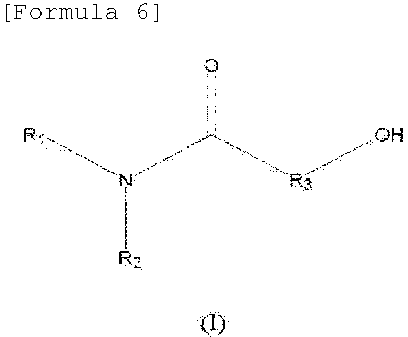 wherein
   R₁ is a C6-C22 hydrocarbon group,
   R₂ is H, or a C6-C22 hydrocarbon group,
   R₃ is a linear or branched C2-C21 hydrocarbon group,
   and a carboxy group-containing polymer,
   and a higher fatty acid.
[17] An agent containing an amide alcohol represented by formula (I) : 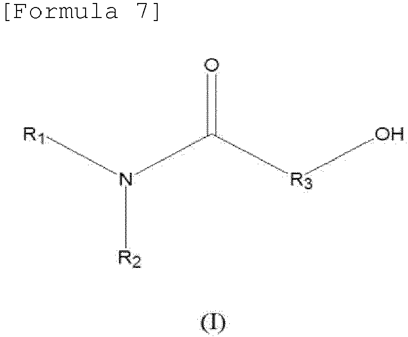 wherein
   R₁ is a C6-C22 hydrocarbon group,
   R₂ is H, or a C6-C22 hydrocarbon group,
   R₃ is a linear or branched C2-C21 hydrocarbon group,
   wherein the agent forms a complex with a carboxy group-containing polymer and is used for W/O type emulsification.
[18] An agent containing an amide alcohol represented by formula (I) : 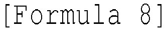 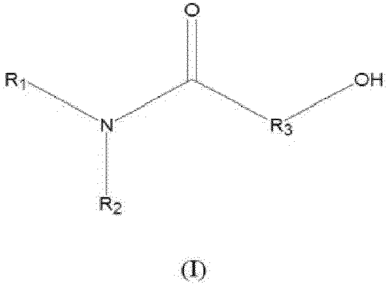 wherein
   R₁ is a C6-C22 hydrocarbon group,
   R₂ is H, or a C6-C22 hydrocarbon group,
   R₃ is a linear or branched C2-C21 hydrocarbon group,
   wherein the agent forms a complex with a carboxy group-containing polymer and is used for W/O type emulsification.
[19] An agent containing a carboxy group-containing polymer, wherein the agent forms a complex with an amide alcohol represented by formula (I): 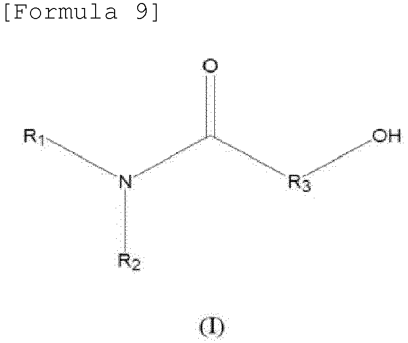 wherein
   R₁ is a C6-C22 hydrocarbon group,
   R₂ is H, or a C6-C22 hydrocarbon group,
   R₃ is a linear or branched C2-C21 hydrocarbon group,
   and is used for W/O type emulsification.
[20] The agent according to [18] or [19], which further forms a complex with a higher fatty acid and is used for W/O type emulsification.
[21] An agent containing a higher fatty acid, which further forms a complex with an amide alcohol represented by formula (I): 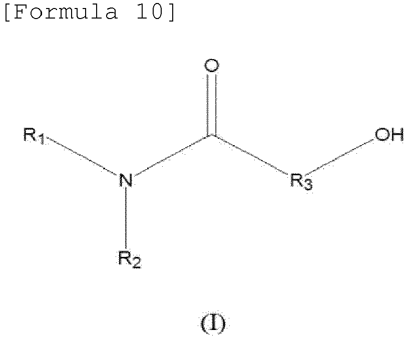 wherein
   R₁ is a C6-C22 hydrocarbon group,
   R₂ is H, or a C6-C22 hydrocarbon group,
   R₃ is a linear or branched C2-C21 hydrocarbon group,
   and a carboxy group-containing polymer, and which is used for W/O type emulsification.

The present invention further relates to [A1] to [A7].
[A1] An O/W emulsified composition comprising a carboxy group-containing polymer, an amide alcohol represented by formula (I) : 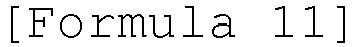 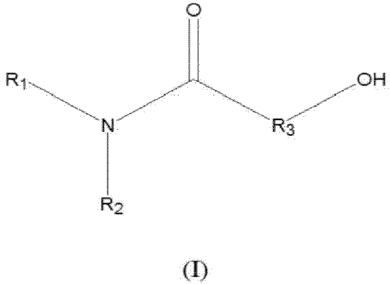 wherein
   R₁ is a C6-C22 hydrocarbon group,
   R₂ is H, or a C6-C22 hydrocarbon group,
   R₃ is a linear or branched C2-C21 hydrocarbon group,
   and one or more polyhydric alcohols.
[A2] The O/W emulsified composition according to [A1], wherein the carboxy group-containing polymer has a weight average molecular weight of 500,000 to 3,000,000 and a carboxy group content of 50 to 70%.
[A3] The O/W emulsified composition according to [A1] or [A2], wherein the polyhydric alcohol is a diol.
[A4] The O/W emulsified composition according to any one of [A1] to [A3], wherein the polyhydric alcohol is pentylene glycol and/or 2-ethyl-1,3-hexanediol.
[A5] The O/W emulsified composition according to any one of [A1] to [A4], further comprising a higher fatty acid.
[A6] A complex used for O/W type emulsification, which is formed by bonding a carboxy group-containing polymer, an amide alcohol represented by formula (I): 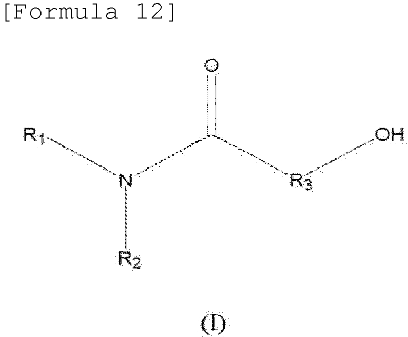 wherein
   R₁ is a C6-C22 hydrocarbon group,
   R₂ is H, or a C6-C22 hydrocarbon group,
   R₃ is a linear or branched C2-C21 hydrocarbon group,
   and one or more polyhydric alcohols.
[A7] The complex used for O/W type emulsification according to [A6], further comprising a higher fatty acid.

### [Advantageous Effects of Invention]

The present invention provides a novel composition and a novel complex that can be used as an emulsifier. The agent and complex of the present invention exert a water-in-oil type or oil-in-water type emulsifying power.

In addition, the polyhydric alcohol can exert a high emulsifying power by hydrogen bonding with a carboxy group-containing polymer, an amide alcohol and a higher fatter acid, thereby providing a water-in-oil or oil-in-water emulsion excellent in stability. Furthermore, the present invention can provide a stable water-in-oil or oil-in-water emulsion without using a surfactant. Moreover, the present invention can provide a water-in-oil or oil-in-water emulsion with excellent feeling of use which can be utilized as cosmetic materials.

### [Brief Description of Drawings]

[Fig. 1] Figure 1 is a diagram showing an emulsified state of Example 1.
[Fig. 2] Figure 2 is a graph showing FT-IR measurement results of amide alcohol, carboxy group-containing polymer, isostearic acid, and blended preparation.
[Fig. 3] Figure 3 is a graph made by superimposing respective FT-IR measurement results.
[Fig. 4] Figure 4 is a diagram showing an emulsified state of Example 2.
[Fig. 5] Figure 5 is a diagram showing an emulsified state of Example 3 and Comparative example 3.
[Fig. 6] Figure 6 is a diagram showing an emulsified state of Example 4 before neutralization and after neutralization.
[Fig. 7] Figure 7 is a diagram showing an emulsified state of Examples 5A and 5B.
[Fig. 8] Figure 8 is a diagram showing an emulsified state of Examples 6A and 6B.
[Fig. 9] Figure 9 is a diagram showing an emulsified state of Examples 7A, 7B and 7C.
[Fig. 10] Figure 10 is a diagram showing an emulsified state of Example 8A (pre-addition) and Example 8B (post-addition).
[Fig. 11] Figure 11 is a diagram showing results of Example 15.
[Fig. 12] Figure 12 is a diagram showing results of Example 16.
[Fig. 13] Figure 13 is a diagram showing results of Example 17.
[Fig. 14] Figure 14 is a diagram showing results of Example 18.

### [Embodiments for Carrying out the Invention]

### Ingredient (A): amide alcohol

The amide alcohol used in the present invention is represented by the following formula (I): 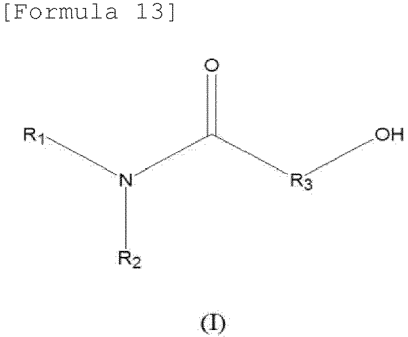 wherein
R₁ is a C6-C22 hydrocarbon group,
R₂ is H, or a C6-C22 hydrocarbon group,
R₃ is a linear or branched C2-C21 hydrocarbon group.

In one embodiment of the present invention, an amide alcohol of formula (I) wherein R₁ is a C10-C22 hydrocarbon group, R₂ is H, and R₃ is a C3-C12 hydrocarbon group is preferred, and an amide alcohol of formula (I) wherein R₁ is a C12-C18 hydrocarbon group, R₂ is H, and R₃ is a C3-C5 hydrocarbon group is particularly preferred.

In another embodiment of the present invention, an amide alcohol of formula (I), wherein R₁ is a linear or branched unsaturated C10-C22 hydrocarbon group; or a cyclic C6-C22 hydrocarbon group; or a benzyl group or phenylethyl group, is preferred.

In a preferred embodiment of the present invention, the amide alcohol of formula (I) has a structure of formulas (I-1) to (1-4) below:

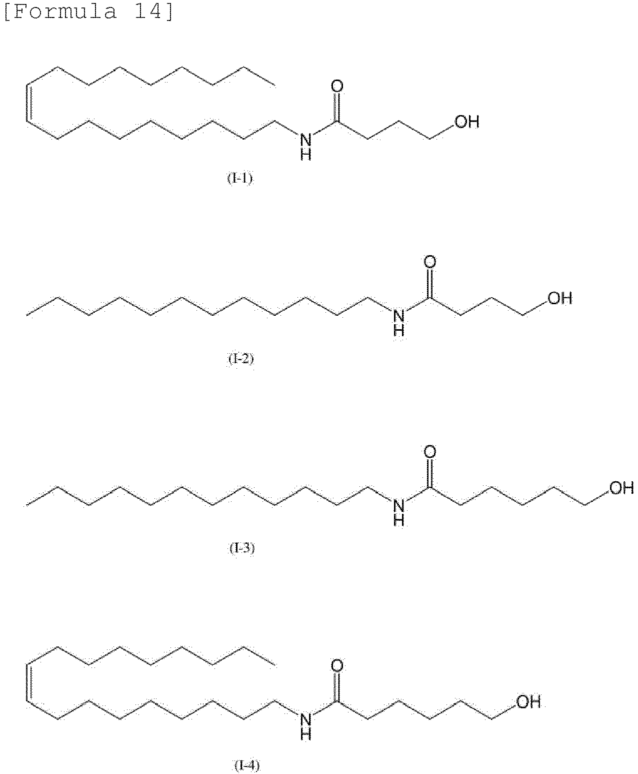

As used herein, the term "hydrocarbon group" may be, unless otherwise specified, saturated or unsaturated, linear, branched or cyclic, or a combination of linear or branched with cyclic and includes, for example, a hydrocarbon group consisting of a linear or branched hydrocarbon moiety and a cyclic hydrocarbon moiety such as benzyl group, phenylethyl group, etc..

That is, the C6-C22 hydrocarbon group in R₁ and R₂ includes a linear, branched or cyclic C6-C22 hydrocarbon group, or a C6-C22 hydrocarbon group consisting of a linear or branched hydrocarbon moiety and a cyclic hydrocarbon moiety, and examples thereof include cyclic groups such as cyclohexyl, decahydronaphthyl, tetrahydrodicyclopentadiene, sterol, phenyl, naphthyl, anthracenyl, etc.; branched alkyl groups such as ethylhexyl, isostearyl, octyldodecyl, etc.; multibranched alkyl groups such as dimethyl, trimethyl , tetramethyl, etc.; linear alkyl groups such as hexyl, octyl, lauryl, myristyl, cetyl, stearyl, aralkyl, behenyl, etc.; and alkenyl groups such as oleyl and elaidyl, etc.

In one embodiment of the invention, R₁ is preferably cyclohexyl, ethylhexyl, octyl, lauryl, myristyl, stearyl, oleyl, benzyl or phenylethyl, with lauryl and oleyl being particularly preferred.

In one embodiment of the present invention, R₂ is preferably H.

The hydrocarbon group in R₃ is a linear or branched C2-C21 hydrocarbon group having no cyclic structure, and examples thereof include alkyl groups such as propyl, butyl, pentyl, hexyl, heptyl, octyl, ethylhexyl, etc., and alkenyl groups such as butylene, pentylene, hexylene, heptylene, etc.

In one embodiment of the present invention, R₃ is preferably propylene, butylene, pentylene or hexylene.

Amide alcohols can be prepared using known synthetic methods.

Examples include:
aminolysis reaction of acid chloride and amine (Schotten-Baumann reaction),
aminolysis reaction of anhydrous fatty acid and amine,
aminolysis reaction of methyl ester and amine,
aminolysis reaction of fatty acid and amine,
aminolysis reaction of lactone and amine,
and the like.

Specifically, for example, it can be synthesized by a method described in JP A No. 2016-114276.

### Ingredient (B): carboxy group-containing polymer

The carboxy group-containing polymer used in the present invention is not particularly limited as long as it is a polymer having a carboxy group in the molecule. From the viewpoint of providing appropriate emulsifying ability, typically, those having a weight average molecular weight of 500,000 to 3,000,000 and a carboxy group content in the molecular weight of approximately 50 to 70% are preferred. "Carboxy group content in the molecular weight of 50 to 70%" means that, of the repeating unit parts which are considered to contain carboxy groups in the basic skeleton of the polymer, carboxy groups are present in 50 to 70% of said repeating unit parts.

The carboxy group-containing polymer becomes water-soluble by neutralization with an alkaline substance, and it is generally used as a thickener.

Examples of carboxy group-containing polymer include carboxyvinyl polymer, and alkyl-modified carboxyvinyl polymers such as alkyl acrylate/methacrylate copolymer, etc., acrylic polymers such as alkyl acrylate/alkyl methacrylate polyoxyethylene ester copolymer, alkyl acrylate/alkyl itaconate polyoxyethylene ester copolymer, steareth-10 allyl ether/alkyl acrylate copolymer, etc., and non-acrylic polymers such as methyl vinyl ether/maleic anhydride/decadiene copolymer, etc.

As the carboxy group-containing polymer, carboxyvinyl polymers and alkyl-modified carboxyvinyl polymers are particularly preferred.

A carboxyvinyl polymer, also called carbomer (INCI name: Carbomer), is a polymer having a structure represented by the following formula (II): 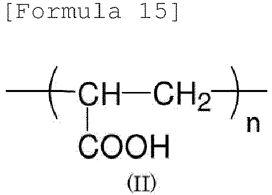 wherein
n is an integer, which is typically from 40 to 100.

Specifically, examples include carboxyvinyl polymers commercially available under the following trade name:
Acritamer 934 (Rita Corporation)
Acritamer 940 (Rita Corporation)
Acritamer 941 (Rita Corporation)
Acritamer 990 (Rita Corporation)
Acritamer 501E (Rita Corporation)
Acritamer 504E (Rita Corporation)
Acritamer 505E (Rita Corporation)
AEC Carbomer 940 (A & E Connock (Perfumery & Cosmetics) Ltd.)
Aqupec HV-501 (Sumitomo Seika Chemicals Co., Ltd.)
Aqupec HV-504 (Sumitomo Seika Chemicals Co., Ltd.)
Aqupec HV-505 (Sumitomo Seika Chemicals Co., Ltd.)
Aqupec HV-501E (Sumitomo Seika Chemicals Co., Ltd.)
Aqupec HV-504E (Sumitomo Seika Chemicals Co., Ltd.)
Aqupec HV-505E (Sumitomo Seika Chemicals Co., Ltd.)
Aqupec HV-801E (Sumitomo Seika Chemicals Co., Ltd.)
Aqupec HV-805E (Sumitomo Seika Chemicals Co., Ltd.)
Aqupec HV-505ED (Sumitomo Seika Chemicals Co., Ltd.)
Aqupec HV-801EG (Sumitomo Seika Chemicals Co., Ltd.)
Aqupec HV-805EG (Sumitomo Seika Chemicals Co., Ltd.)
Carbopol Clear Polymer (Lubrizol Advanced Materials, Inc.)
Carbopol ETD 2050 Polymer (Lubrizol Advanced Materials, Inc.)
Carbopol 934 Polymer (Lubrizol Advanced Materials, Inc.)
Carbopol 940 Polymer (Lubrizol Advanced Materials, Inc.)
Carbopol 941 Polymer (Lubrizol Advanced Materials, Inc.)
Carbopol 980 Polymer (Lubrizol Advanced Materials, Inc.)
Carbopol 981 Polymer (Lubrizol Advanced Materials, Inc.)
Carbopol 2984 Polymer (Lubrizol Advanced Materials, Inc.)
Carbopol 5984 Polymer (Lubrizol Advanced Materials, Inc.)
Carbopol Ultrez 10 Polymer (Lubrizol Advanced Materials, Inc.)
Carbopol Ultrez 30 Polymer (Lubrizol Advanced Materials, Inc.)
CustoPoly J 100 (Custom Ingredients, Inc.)
CustoPoly J 300 (Custom Ingredients, Inc.)
CustoPoly J 400 (Custom Ingredients, Inc.)
Easygel DO (3V Sigma USA Inc.)
Flogel 700 (SNF SAS)
Flogel 1000 (SNF SAS)
Junlon PW-110 (Nihon Junyaku Company, Ltd.)
Junlon PW-111 (Nihon Junyaku Company, Ltd.)
Junlon PW-302S (Nihon Junyaku Company, Ltd.)
Polacril 40 (Lehvoss Italia S.r.l.)
Polygel CA (3V Sigma USA Inc.)
Polygel CB (3V Sigma USA Inc.)
Polygel CS (3V Sigma USA Inc.)
Polygel DV (3V Sigma USA Inc.)
Polygel TG (3V Sigma USA Inc.)
SuperGel CE (Sino Lion USA)
Synthalen K (3V Sigma USA Inc.)
Synthalen L (3V Sigma USA Inc.)
Synthalen M (3V Sigma USA Inc.)
Tego Carbomer 134 (Evonik Nutrition & Care GmbH)
Tego Carbomer 140 (Evonik Nutrition & Care GmbH)
Tego Carbomer 141 (Evonik Nutrition & Care GmbH)
Tego Carbomer 340 FD (Evonik Nutrition & Care GmbH).

The alkyl-modified carboxyvinyl polymer is a copolymer of acrylic acid and/or methacrylic acid with alkyl acrylate and/or alkyl methacrylate thereof.

Specific examples of alkyl-modified carboxyvinyl polymer include acrylates/alkyl acrylates (C10-30)) cross polymer (INCI name: Acrylates/C10-30 Alkyl Acrylates Cross polymer, also called alkyl acrylate/methacrylate copolymer), which is a polymer having a structure represented by the following formula (III): 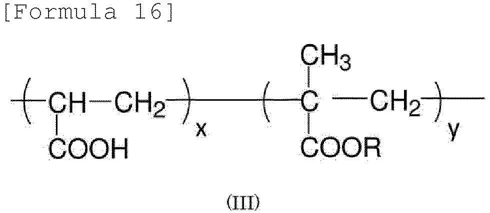 wherein
R is a C10-30 alkyl group,
x and y are integers, each of which can be arbitrarily selected from integers of 1 or more, and typically x + y = 40 to 100, and when y is 2 or more, R may be the same or different.

Specifically, the examples include alkyl-modified carboxyvinyl polymers commercially available under the following trade name:
Acritamer 501ED (Rita Corporation)
Acritamer 505ED (Rita Corporation)
Aqupec HV-701EDR (Sumitomo Seika Chemicals Co., Ltd.)
Aqupec HV-501ER (Sumitomo Seika Chemicals Co., Ltd.)
Aqupec SER W-150C (Sumitomo Seika Chemicals Co., Ltd.)
Aqupec SER W-300C (Sumitomo Seika Chemicals Co., Ltd.)
Carbopol ETD 2020 Polymer (Lubrizol Advanced Materials, Inc.)
Carbopol 1342 Polymer (Lubrizol Advanced Materials, Inc.)
Carbopol 1382 Polymer (Lubrizol Advanced Materials, Inc.)
Carbopol SC 200 (Lubrizol Advanced Materials, Inc.)
Carbopol SC 500 Polymer (Lubrizol Advanced Materials, Inc.)
Carbopol Ultrez 20 Polymer (Lubrizol Advanced Materials, Inc.)
Carbopol Ultrez 21 Polymer (Lubrizol Advanced Materials, Inc.)
Carbopol Xtra-11 Polymer (Lubrizol Advanced Materials, Inc.)
Pemulen EZ-4U Polymeric Emulsifier (Lubrizol Advanced Materials, Inc.)
Pemulen TR-1 Polymer (Lubrizol Advanced Materials, Inc.) Pemulen TR-2 Polymer (Lubrizol Advanced Materials, Inc.)
Tego Carbomer 341 ER (Evonik Nutrition & Care GmbH)
TEGO Carbomer 750 HD (Evonik Nutrition & Care GmbH).

### Ingredient (C): Polyhydric alcohol

The polyhydric alcohol used in the present invention is not particularly limited as long as it is a compound having a plurality of hydroxy groups in the molecule; however, from the viewpoint of providing appropriate emulsifying ability, typically it is a polyhydric alcohol of 3 to 10 carbon atoms, preferably 5 to 8 carbon atoms. Also, the number of hydroxy groups in the molecule of polyhydric alcohol is 2 to 4, and preferably 2.

Specific examples of polyhydric alcohols include pentylene glycol, 2-ethyl-1,3-hexanediol, 1,2-pentanediol, 1,3-butylene glycol, propane-1,2,3-triol, propane-1,2-diol, 1,2-octanediol, 1,2-hexanediol, etc., and preferably, pentylene glycol and 2-ethyl-1,3-hexanediol.

The polyhydric alcohols can be used alone or in combination of two or more kinds.

The content of polyhydric alcohol is 0.1 to 20 wt%, preferably 1.0 to 15 wt%, and most preferably 3.0 to 10 wt%.

The polyhydric alcohol is expected to contribute to the stabilization of a complex and the improvement of emulsifying power by hydrogen bonding with an amide alcohol, a carboxy group-containing polymer, and/or a higher fatty acid.

### <Higher fatty acid>

Higher fatty acid used in the present invention is not particularly limited as long as it is an ingredient generally used in cosmetics and the like. Examples include a higher fatty acid represented by the general formula (G):

R₃COOH (G)

wherein R₃ is a linear or branched, saturated or unsaturated hydrocarbon having an average carbon number of 7 to 25 which may have a hydroxyl group, and these may be used alone or in combination of two or more kinds.

From the viewpoint of forming a complex suitable for emulsification, a higher fatty acid wherein R₃ in the above general formula (G) is a linear or branched chain having an average carbon number of 11 to 21 is preferred.

Specific examples include saturated fatty acids such as lauric acid, myristic acid, palmitic acid, stearic acid, arachic acid, behenic acid, etc.; unsaturated fatty acids such as 2-palmitoleic acid, petroselinic acid, oleic acid, elaidic acid, ricinoleic acid, linoleic acid, linoelaidic acid, linolenic acid and arachidonic acid, etc., branched fatty acids such as isostearic acid, etc.; hydroxycarboxylic acids such as 12-hydroxystearic acid, etc. Of these, from the viewpoints of stability and skin irritation, saturated fatty acids having a carbon number of 18 are preferable, and among them, those having branching are preferable, and saturated fatty acids with a carbon number of 18 having methyl branching are more preferable. Examples of commercially available products include isostearic acid (trade name: Isostearic acid EX, HAIMARIC-MKH(R), manufactured by Kokyu Alcohol Kogyo Co., Ltd.), SR PRISORINE 3505 (Croda Japan, K.K.), Isostearic acid, Isostearic acid T, (manufactured by Nissan Chemical Corporation) and the like.

The higher fatty acid is considered to function as a part of the complex in addition to the function as an oil agent in the emulsion.

In one embodiment of the present invention, "complex" means a complex formed from an amide alcohol and one or more kinds selected from the group consisting of a carboxy group-containing polymer, a polyhydric alcohol, and a higher fatty acid.

In W/O emulsions, the complex can be formed by adding a carboxy group-containing polymer to an oil phase part containing an amide alcohol and a higher fatty acid, and the complex with higher emulsifying power can be formed by further neutralizing with an alkali. In another embodiment of the present invention, in W/O emulsions, the complex can be formed by adding an aqueous phase part containing a carboxy group-containing polymer and a polyhydric alcohol to an oil phase part containing an amide alcohol and a higher fatty acid.

In O/W emulsions, the complex with high emulsifying power can be formed by adding an oil phase containing an amide alcohol and a higher fatty acid to an aqueous phase part containing a carboxy group-containing polymer and a polyhydric alcohol, and then neutralizing with an alkali.

In the emulsion according to the present invention, the emulsifying ability is considered to be exerted as follows: the amide bond portion of the amide alcohol and the carbonyl group of the polymer and the higher fatty acid form a hydrogen bond, and a part of the carboxy group becomes COO⁻ to have hydrophilicity. Furthermore, in one embodiment of the present invention, the emulsifying ability is considered to be exerted as follows: the amide bond portion of the amide alcohol, the hydroxy group of the polyhydric alcohol, and the carboxy group of the carboxy group-containing polymer and the higher fatty acid form a hydrogen bond, and a part of the carboxy group becomes COO⁻ to have hydrophilicity.

Therefore, without being bound by any theory, the complex of the present invention is considered to be formed by bonding, more specifically hydrogen bonding the amide bond portion of the amide alcohol with the carbonyl group of the polymer and the carbonyl group of the higher fatty acid; or the amide bond portion of the amide alcohol with at least one member selected from the group consisting of hydroxy group of the polyhydric alcohol, carbonyl group of the carboxy group-containing polymer, and carboxy group of the higher fatty acid as a partner.

In the present invention, "emulsion" means, unless otherwise specified, both W/O emulsion and O/W emulsion.

In the present invention, "W/O emulsion" is a water-in-oil emulsion, that is an emulsion in which an aqueous ingredient is dispersed in a continuous phase comprising an oily ingredient.

W/O emulsions can be prepared by dispersing an aqueous phase comprising a carboxy group-containing polymer in an oil phase comprising an amide alcohol, and neutralizing with an alkali.

In the present invention, "O/W emulsion" is an oil-in-water emulsion, that is, an emulsion in which an oily ingredient is dispersed in a continuous phase comprising an aqueous ingredient.

O/W emulsions can be prepared by dispersing an oil phase comprising an amide alcohol in an aqueous phase comprising a carboxy group-containing polymer, and neutralizing with an alkali.

In one embodiment of the present invention, emulsions can be used for all purposes, but typically, they can be used for external preparations such as pharmaceuticals, quasi drugs, and cosmetics, etc.

The emulsion of the present invention can be used for various forms of products including pharmaceuticals such as external skin preparation comprising a drug; quasi drugs such as medicated cosmetics; skin care cosmetics such as gel lotion, milky lotion, cream, beauty essence, sunscreen, and daytime moisturizer; makeup cosmetics such as foundation, makeup base, eye shadow, mascara, as well as hair care cosmetics such as hair treatment, etc.

### <Oily ingredient>

The oily ingredient used in the emulsion of the present invention is not particularly limited as long as it is a ingredient generally used for cosmetics and the like; and examples thereof include oil agents such as animal and vegetable fats and oils, hydrocarbon oils, higher fatty acids, higher alcohols, ester oils, silicone oils, etc., which can be used alone or in combination of two or more kinds.

Examples of animal and vegetable fats and oils or hydrogenated animal and vegetable fats and oils include avocado oil, perilla oil, olive oil, cacao butter, kaya oil, apricot kernel oil, hydrogenated oil, wheat germ oil, sesame oil, rice germ oil, rice bran oil, sugar cane wax, Camellia sinensis leaf oil, safflower oil, shea butter, Chinese tung oil, cinnamon oil, soybean oil, tea seed oil, camellia oil, evening primrose oil, corn oil, rapeseed oil, germ oil, palm oil, palm kernel oil, castor oil, hydrogenated castor oil, sunflower oil, grape oil, jojoba oil, macadamia nut oil, beeswax, cottonseed oil, cotton wax, Japan wax, montan wax, coconut oil, hardened coconut oil, peanut oil, lanolin, liquid lanolin, reduced lanolin, lanolin alcohol, hard lanolin, lanolin acetate, lanolin fatty acid isopropyl ester, hexyl laurate, etc.

Examples of hydrocarbon oils include ozokerite, squalane, squalene, ceresin, paraffin, isoparaffin, paraffin wax, liquid paraffin (mineral oil), pristane, polyisobutylene, polyisobutene, hydrogenated polyisobutene, microcrystalline wax, polyethylene wax, Vaseline, etc.

Examples of higher fatty acids include lauric acid, myristic acid, palmitic acid, stearic acid, isostearic acid, oleic acid, behenic acid, undecylenic acid, oleic acid, linoleic acid, linolenic acid, arachidonic acid, eicosapentaenoic acid (EPA), docosahexaenoic acid (DHA), etc.

Examples of higher alcohols include myristyl alcohol, cetanol, stearyl alcohol, arachidyl alcohol, behenyl alcohol, hydrogenated rapeseed oil alcohol, etc.

Examples of ester oils include, as monoester, isononanoic acid esters such as isononyl isononanoate, isodecyl isononanoate, isotridecyl isononanoate, tricyclodecanemethyl isononanoate, ethylhexyl isononanoate, etc., 2-ethylhexanoic acids such as cetyl ethylhexanoate, hexyldecyl ethylhexanoate, etc., myristic acid esters such as isopropyl myristate, isocetyl myristate, octyldodecyl myristate, etc., isostearic acid esters such as ethyl isostearate, isopropyl isostearate, hexyldecyl isostearate, isostearyl isostearate, cholesteryl isostearate, phytosteryl isostearate, etc., lactic acid esters such as isostearyl lactate, octyldodecyl lactate, etc., oleic acid esters such as oleyl oleate, phytosteryl oleate, octyldodecyl oleate, etc., neopentanoic acid esters such as isodecyl neopentanoate, isostearyl neopentanoate, etc., palmitic acid esters such as isopropyl palmitate, ethylhexyl palmitate, etc., and others such as octyldodecyl neodecanoate, octyldodecyl ricinoleate, oleyl erucate, octyldodecyl erucate, isopropyl lauroyl sarcosinate, etc.

Examples of diester oils include diisobutyl adipate, diisopropyl adipate, diethylhexyl succinate, neopentyl glycol diisononanoate, neopentyl glycol diethylhexanoate, neopentyl glycol dicaprate, diisostearyl malate, diisopropyl dilinoleate, ethylene glycol dioctanoate, octyldodecyl stearoyl oxystearate, diisopropyl sebacate, di(cholesteryl/octyldodecyl) lauroyl glutamate, di(phytosteryl/octyldodecyl) lauroyl glutamate, etc.

Examples of triester oils include triethylhexanoin, trimethylolpropane triethylhexanoate, glyceryl tri(caprylate/caprate), triisostearin, trimethylolpropane triisostearate, etc.

Examples of tetraester oils include pentaerythrityl tetraethylhexanoate, pentaerythrityl tetraisostearate, etc.

Examples of polyester oils include polyglyceryl fatty acid esters such as polyglyceryl-2 isostearate, polyglyceryl-2 diisostearate, polyglyceryl-2 triisostearate, polyglyceryl-2 tetraisostearate, etc.

Examples of highly viscous ester oils include hydrogenated castor oil isostearate, hydrogenated castor oil dimer dilinoleate, (polyglyceryl-2 isostearate/dimer dilinoleate) copolymer, (phytosteryl/isostearyl/cetyl/stearyl/behenyl) dimer dilinoleate, dimer dilinoleyl bis(phytosteryl/behenyl/isostearyl) dimer dilinoleate, di(isostearyl/phytosteryl) dimer dilinoleate, dimer dilinoleyl hydrogenated rosin condensate, dimer dilinoleyl diisostearate, dimer dilinoleyl dimer dilinoleate, di(cholesteryl/behenyl/octyldodecyl) lauroyl glutamate, di(octyldodecyl/phytosteryl/behenyl) lauroyl glutamate, myristoyl methyl alanine (phytosteryl/decyltetradecyl), etc.

Examples of silicone oils include dimethylpolysiloxane, methyl phenyl polysiloxane, alkyl-modified organopolysiloxane, terminal modified organopolysiloxane, fluorine-modified organopolysiloxane, amodimethicone, amino-modified organopolysiloxane, volatile silicone, alkyl dimethicone, cyclopentasiloxane, etc.

In one embodiment of the present invention, the blending amount of the oily ingredient in the W/O emulsion is not particularly limited; however, from the viewpoint of usability, it may be 30.0 to 80.0 mass%, preferably 40.0 to 70.0 mass%, more preferably 50.0 to 60.0 mass%.

In one embodiment of the present invention, the blending amount of the oily ingredient in the O/W emulsion is not particularly limited; however, from the viewpoint of usability, it may be 1.0 to 40.0 mass%, preferably 3.0 to 30.0 mass%, more preferably 5.0 to 20.0 mass%.

In the present invention, from the viewpoint of providing an emulsion that exhibits stability for a longer period of time, it is preferable that the ratio of nonpolar oils such as hydrocarbon oil and silicone oil in the oily ingredient is high.

From the viewpoint of obtaining a long-term stable emulsion without using a surfactant, it is preferable that the mass ratio of nonpolar oil/(nonpolar oil + polar oil) exceeds 0.5.

### <Aqueous ingredient>

Aqueous ingredients used in the emulsion of the present invention are not particularly limited as long as they are ingredients generally used in cosmetics and the like; and examples thereof include water such as purified water, ion exchanged water, etc.; and lower alcohols such as BG (1,3-butylene glycol), PG (propylene glycol), glycerin, ethanol, etc., and these can be used alone or in combination of two or more kinds.

In one embodiment of the present invention, the blending amount of the aqueous ingredient in the W/O emulsion is not particularly limited; from the viewpoint of usability, it may be 10.0 to 70.0 mass%, preferably 15.0 to 65.0 mass%, more preferably 20.0 to 60.0 mass%.

In one embodiment of the present invention, the blending amount of the aqueous ingredient in the O/W emulsion is not particularly limited; from the viewpoint of usability, it may be 50.0 to 90.0 mass%, preferably 55.0 to 85.0 mass%, more preferably 60.0 to 80.0 mass%.

### <Neutralizing agent>

A neutralizing agent used for preparing the emulsion of the present invention is not particularly limited as long as it is an alkaline ingredient generally used in cosmetics and the like, and examples thereof include potassium hydroxide, triethanolamine, sodium hydroxide, basic amino acids such as L-arginine and L-lysine, 2-amino-2-methyl-1-propanol, etc., and these can be used alone or in combination of two or more kinds.

The blending amount of a neutralizing agent can be appropriately selected depending on the type of the neutralizing agent and the composition of the whole emulsion, and it is typically about 0.01 to 1.0 mass%.

In addition, the neutralizing agent may be an active ingredient such as tranexamic acid, carnosine, etc.

### <Surfactant>

In the present specification, the term "surfactant" means a compound having both a hydrophilic group and a hydrophobic group in one molecule, and a surfactant may be appropriately added as necessary to emulsions.

In one embodiment of the present invention, because the complex of the present invention has an emulsifying ability, preferably emulsions are substantially free of surfactant. This makes it possible to provide an emulsifier with less irritation.

Here, "substantially free of" means that a surfactant is not contained in an amount sufficient for the emulsification of the whole emulsion. In addition, in the present invention, "substantially free of surfactant" means that it comprises no surfactant at all or comprises a surfactant in an amount that is not emulsified. The amount that is not emulsified can be appropriately determined by a person skilled in the art according to the compositional ratio, for example, in one embodiment it is less than 2.0 mass%, in another embodiment it is less than 0.2 mass%, or less than 0.02 mass%.

In a particular embodiment of the invention, the conditioning composition is an emulsified composition substantially free of surfactant such as cationic surfactant.

### <Other ingredients>

The emulsion of the present invention may comprise any ingredients used in external preparations such as cosmetics, etc.

Examples of these additional ingredients include ultraviolet absorbers such as ethylhexyl methoxycinnamate, Diethylamino hydroxybenzoyl hexyl benzoate, etc.; thickeners and gelling agents such as dextrin palmitate, xanthan gum, etc.; quality maintaining ingredients such as antioxidant, preservative, etc.; skin softeners (emollients); medicinal ingredients and active ingredients such as whitening agent, anti-wrinkle agent, antioxidative agent, etc.; perfumes, coloring agents such as pigment and dyestuff, and the like.

In one embodiment of the present invention, in order to improve stability, the outer oil layer is oil-gelled with an oil gelling agent in the W/O emulsion, or the inner oil layer is oil-gelled with an oil gelling agent in the O/W emulsion.

As an ingredient used for oil-gelling, an organically modified clay mineral and/or a polymer ester of sugar and fatty acid can be used.

The organically modified clay mineral and the polymer ester of sugar and fatty acid used in the preparation of emulsions of the present invention are not particularly limited as long as they are ingredients generally used in cosmetics and the like.

Examples of organically modified clay mineral include water-swellable clay minerals (for example, montmorillonite, saponite, hectorite, bentonite, etc.) in which a convertible cation interposed between crystal layers is replaced with an organic polar compound or an organic cation (for example, a quaternary ammonium salt-type cationic surfactant).

In the present invention, organically modified clay minerals which are commercially available under the trade names of, for example, BENTONE 27V (stearalkonium hectorite), BENTONE 27VCG (stearalkonium hectorite), BENTONE 38V (disteardimonium hectorite), BENTONE 38VCG (disteardimonium hectorite), etc. from Elementis Specialties Inc. can be used.

In addition, organically modified clay minerals are also commercially available as a premixed product dissolved in silicone oil, ester oil and/or other oil agent; in the present invention, such a premix may be used, and for example, it is commercially available from Elementis Specialties Inc. under the following trade names:
BENTONE GEL 1002V (cyclopentasiloxane, disteardimonium hectorite, propylene carbonate), BENTONE GEL ABO V (Crambe abyssinica seed oil, stearalkonium hectorite, propylene carbonate), BENTONE GEL CAO V (castor oil, stearalkonium hectorite, propylene carbonate), BENTONE GEL EUG V (octyldodecanol, disteardimonium hectorite, propylene carbonate), BENTONE GTCC V (glyceryl tri(caprylate/caprate), stearalkonium hectorite, propylene carbonate), BENTONE HSO V (glyceryl tri(caprylate/caprate), stearalkonium hectorite, propylene carbonate), BENTONE IHD V (isohexadecane, disteardimonium hectorite, propylene carbonate), BENTONE IPM V (isopropyl myristate, stearalkonium hectorite, propylene carbonate), BENTONE ISD V (isododecane, disteardimonium hectorite, propylene carbonate), BENTONE LOI V (liquid lanolin, isopropyl palmitate, stearalkonium hectorite, propylene carbonate), BENTONE MSO(V) (meadowfoam oil, disteardimonium hectorite, propylene carbonate), BENTONE NGD V (neopentyl glycol diheptanoate, disteardimonium hectorite, propylene carbonate), BENTONE OLV V (olive fruit oil, stearalkonium hectorite, propylene carbonate), BENTONE OMS V ((C11, 12) isoparaffin, disteardimonium hectorite, denatured alcohol), BENTONE PTIS V (pentaerythrityl tetraisostearate, disteardimonium hectorite, propylene carbonate), BENTONE SS71V (petroleum volatiles, disteardimonium hectorite, propylene carbonate), BENTONE TMF V (methyl trimethicone, disteardimonium hectorite, triethyl citrate), BENTONE TNV ((alkyl (C12-15) benzoate, stearalkonium hectorite, propylene carbonate), BENTONE VS-5V(V) (cyclopentasiloxane, disteardimonium hectorite, denatured alcohol), BENTONE VS-5PC V(HV) (cyclopentasiloxane, disteardimonium hectorite, propylene carbonate).

The blending amount of the organically modified clay mineral in the W/O type emulsion can be appropriately adjusted depending on the kind and amount of the oily ingredient and the viscosity required for the emulsion, and is not particularly limited; it may be 0.5 to 8.0 mass%, preferably 0.7 to 6.0 mass%, more preferably 1.0 to 5.0 mass%.

The blending amount of the organically modified clay mineral in the O/W emulsion can be appropriately adjusted depending on the kind and amount of the oily ingredient and the viscosity required for the emulsion, and is not particularly limited; however, it may be 0.1 to 3.0 mass%, preferably 0.1 to 2.0 mass%, more preferably 0.1 to 1.0 mass%.

Examples of polymer esters with sugar and fatty acid include those commercially available under the following trade names by Chiba Flour Milling Co. Ltd.:
Rheopearl KL (dextrin palmitate), Rheopearl TL2 (dextrin palmitate), Rheopearl MKL (dextrin myristate), Rheopearl TT2 [dextrin (palmitate/ethylhexanoate)], Rheopearl WX [dextrin (palmitate/hexyldecanoate)], Rheopearl ISL2 (inulin stearate), Rheopearl ISK2 (inulin stearate).

The blending amount of the polymer ester with sugar and fatty acid in the W/O emulsion can be appropriately adjusted depending on the kind and amount of the oily ingredient and the viscosity required for the emulsion, and is not particularly limited; it may be 0.5 to 8.0 mass%, preferably 0.7 to 6.0 mass%, more preferably 1.0 to 5.0 mass%.

The blending amount of the polymer ester with sugar and fatty acid in the O/W emulsion can be appropriately adjusted depending on the kind and amount of the oily ingredient and the viscosity required for the emulsion, and is not particularly limited; it may be 0.1 to 3.0 mass%, preferably 0.1 to 2.0 mass%, more preferably 0.1 to 1.0 mass%.

In addition, in one embodiment of the present invention, by blending a polyhydric alcohol such as pentylene glycol, octylene glycol etc., emulsifying properties as an emulsion can be further improved.

Without being bound by any theory, it is considered that improvement of emulsifying properties by blending a polyhydric alcohol is achieved by the formation of a complex by hydrogen bonding of a hydroxyl group in the molecule of the polyhydric alcohol and a carbonyl group of the amide bond in the amide alcohol molecule.

Accordingly, the present invention also provides a complex formed from an amide alcohol, a carboxy group-containing polymer, a higher fatty acid and a polyhydric alcohol.

It is to be noted that pentylene glycol has a moisturizing action and an antibacterial action, and it is a preferable ingredient for using emulsion as an external preparation such as cosmetics.

Specifically, pentylene glycol is commercially available under the following trade names:
Diol PD (Kokyu Alcohol Kogyo Co., Ltd.) and Diol PD-V (Kokyu Alcohol Kogyo Co., Ltd.).

Specifically, octylene glycol is commercially available under the following trade name:
OD-eight (Kankohsha Co., Ltd.).

The blending amount of the ingredient (A) in the emulsion can be appropriately selected depending on the type and amount of the oil agent to be used and the viscosity required, etc., and it is typically 0.1 to 15. 0 mass%, preferably 0.5 to 10.0% mass%, more preferably 1.0 to 8.0 mass%.

The blending amount of the ingredient (B) in the emulsion can be appropriately selected depending on the type and amount of the oil agent to be used, the viscosity required for the emulsified composition, etc. Typically, it is 0.01 to 5.0 mass%, preferably 0.05 to 3.0 mass%, more preferably 0.1 to 2.0 mass%.

By using the above blending amounts, an amount sufficient to obtain an emulsified composition having good usability and stability of the complex consisting of ingredient A and ingredient B in the present invention is realized.

In an emulsified composition, the ratio of wt% of amide alcohol (ingredient (A)) and carboxy group-containing polymer (ingredient (B)) in the emulsified composition is preferably amide alcohol/carboxy group-containing polymer = 10 to 110, more preferably 15 to 100, still more preferably 20 to 80.

By using these ratios, the balance of hydrophilicity/hydrophobicity becomes suitable for emulsions, and it is possible to provide an emulsion with high stability having fine emulsion particles.

Furthermore, a higher fatty acid may be added to the emulsion. The blending amount of the higher fatty acid can be appropriately selected depending on the type and amount of the oil agent to be used, the viscosity required, etc., and it is typically 0.1 to 7.0 mass%, and more preferably 0.5 to 5.0 mass% in W/O type emulsions, and it is typically 0.1 to 3.0 mass%, and more preferably 0.1 to 2.0 mass% in O/W type emulsions.

By using the above blending amounts, the amounts suitable for obtaining an emulsified composition having good usability and stability of the water-in-oil type of complex consisting of the amide alcohol, carboxy group-containing polymer and higher fatty acid in the present invention are realized.

The pH of the emulsion of the present invention can be appropriately selected, and in the W/O type emulsion, it is preferably about pH 5.0 to 10.0, more preferably about pH 5.0 to 8.0; and in the O/W type emulsion, it is preferably about pH 5.0 to 8.0, more preferably about pH 5.0 to 7.0. Without being bound by any theory, it is considered that, by setting the pH within this range, the ingredient A and the ingredient B (and the higher fatty acid) are moderately hydrogen bonded to form a complex, and the dissociation of the carboxy group becomes moderate, leading to excellent emulsifying ability.

Viscosity of the emulsion of the present invention can be appropriately selected depending on the characteristics of objective products.

In one embodiment of the present invention, from the viewpoint of obtaining a creamy emulsion, it is preferable to have a viscosity higher than 10,000 mPa·s, and from the viewpoint of obtaining an emulsion in the form of milky lotion, it is preferable to have a viscosity of about 300 to 100,000 mPa·s.

In one embodiment of the present invention, from the viewpoint of using emulsion as a cosmetic material, the viscosity of the emulsion can be appropriately selected within the range of 300 to 1,000,000 mPa·s.

The present invention also provides an agent used for emulsification, containing an amide alcohol, a carboxy group-containing polymer, or a higher fatty acid. Such an agent can be used in the production of emulsions.

Hereinafter, the present invention will be described in more detail based on examples; however, the present invention is not limited to these examples, and various modifications can be made without departing from the technical idea of the present invention. In the present specification, unless otherwise specified, % means mass%.

### [Example]

### <W/O type emulsion>

### Example 1

### [Table 1]

**Table 1: Formulation using carboxy group-containing polymer and amide alcohol**

| Classification | | Material name | Example 1 |
|---|---|---|---|
| Aqueous phase | Base | (1) Ion exchanged water | 38.0 |
| | Humectant | (2) Glycerin | 5.0 |
| | | (3) 1,3-Butylene glycol | 3.0 |
| | Ingredient B | (4) PEMULEN TR-2 | 0.1 |
| Oil phase | Oil agent | (5) Squalane | 48.4 |
| | Ingredient A | (6) Amide alcohol OLH | 5.0 |
| Neutralizing agent | | (7) Potassium hydroxide (10%) | 0.5 |
| Total | | | 100.0 |
| Emulsion particle size (µm) | | | ∼ 30 (50) |
| Viscosity (mPa·s) (No.64, 12 rpm) | | | 9700 |

Regarding the expression of emulsion particle size, "∼30(50)" means that it is on average 30 µm or less, but there are particles with a diameter of about 50 µm scattered in some places. Hereinafter, it is expressed in the same meaning.

As the ingredient A, an amide alcohol of formula (I) wherein R₁ is unsaturated C18 alkyl (oleyl), R₂ is H, R₃ is C5, which has the following structure (1-4) is used.

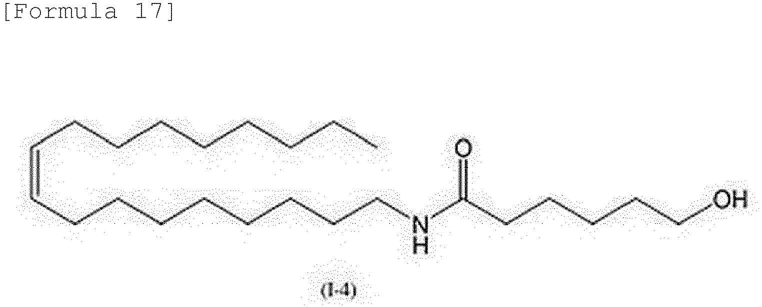

In the present specification, the above amide alcohol is also referred to as "amide alcohol OLH".

As the ingredient B, PEMULEN TR-2 ((acrylates/alkyl acrylate (C10-30)) crosspolymer) was used.

### (Production method)

(1) to (4) were uniformly stirred and dissolved at 80°C to obtain an aqueous phase. Meanwhile, (5) and (6) were uniformly stirred and dissolved at 80°C to obtain an oil phase. The obtained aqueous phase and oil phase were mixed and uniformly dissolved, and the mixture was stirred at 80°C with a disperser to prepare a mixture. While stirring the mixture, (7) was added to obtain a water-in-oil emulsified composition.

### (Evaluation)

### 1. Measurement of emulsion particle size by microscopic observation

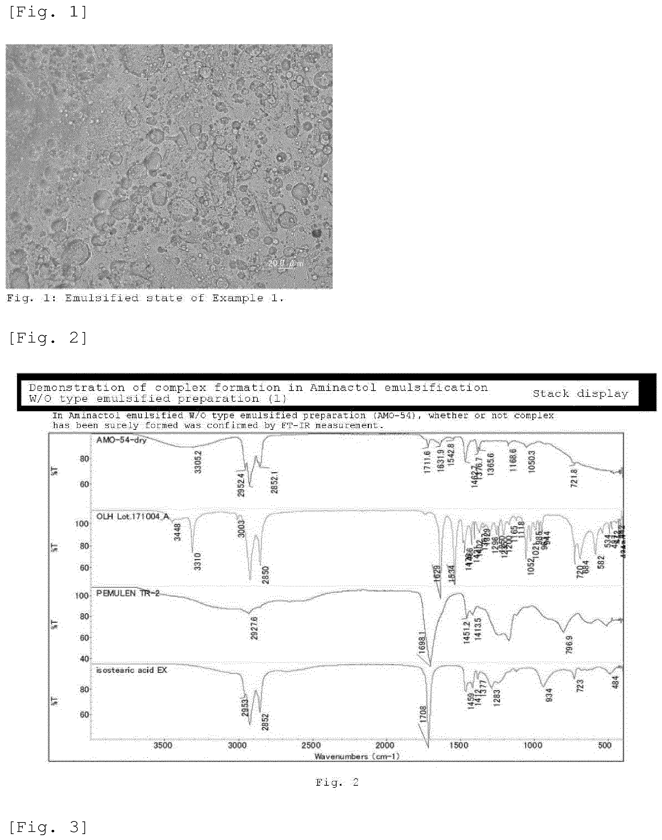

Observation was carried out using "BX-51" manufactured by Olympus Corporation at 400 times magnification, and the emulsion particle size of the emulsion after neutralization was respectively evaluated. The results are shown in Fig. 1.

### 2. Viscosity

Viscosity was measured with Brookfield viscometer, using two types of SPINDLE S63 and S64 depending on the viscosity at a rotation speed of 12 rpm. The viscosity was 942,000 mPa·s. It can be said that an emulsion having a viscosity suitable for a creamy cosmetic composition, etc. can be provided.

### Example 2

In order to observe the emulsion of the basic formulation, the formulation (AMO-54) shown in the following Table 2 was prepared.

### [Table 2]

**Table 2: Basic formulation**

| Material name | | Example 2 |
|---|---|---|
| Aqueous phase | Ion exchanged water | 34.9 |
| | Glycerin | 5.0 |
| | 1,3-Butylene glycol | 3.0 |
| | | |
| | PEMULEN TR-2 | 0.1 |
| | | |
| Oil phase | Squalane | 45.5 |
| | | |
| | Amide alcohol OLH | 5.0 |
| | Isostearic acid EX | 6.0 |
| | | |
| Neutralizing agent | Potassium hydroxide (10%) | 0.5 |
| Total | | 100.0 |
| | | |
| Emulsion particle size (µm) | | ∼30(40) |
| Viscosity (mPa·s) | | 15297 (No.64, 12 rpm) |

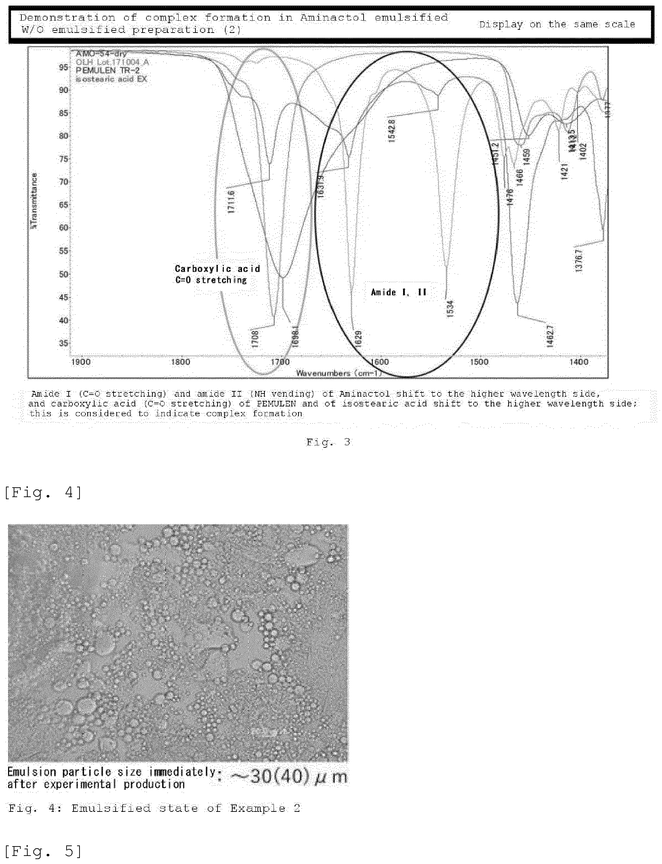

Amide alcohol, carboxy group-containing polymer and isostearic acid were measured with a Fourier transform infrared spectrometer (hereinafter referred to as FT-IR) using an infrared spectroscopic analyzer (Nicolet IS 10, manufactured by Thermo Fisher Scientific Inc.). Measurement results of the amide alcohol (OLH), carboxy group-containing polymer (trade name: PEMULEN TR-2 Polymer, Lubrizol Advanced Materials, Inc.), isostearic acid (trade name: Isostearic acid EX, Kokyu Alcohol Kogyo Co., Ltd.) and the W/O emulsified composition according to the present invention (Example 2 (AMO-54, product dried for 2 weeks at normal temperature)) are shown in Fig. 2, and Fig. 3 shows the result of enlarging the part of 1300 to 2000 cm⁻¹.

Since O-H stretching (3300 to 2500 cm⁻¹) and O-H bending (1000 to 850 cm⁻¹), etc. by a water molecule mask the absorption of complex formation, natural drying of the W/O emulsified composition was a treatment performed to eliminate the absorption by water molecules as much as possible.

From these results, it can be understood that the absorption spectrum of the complex is different from superimposed absorption spectra of the amide alcohol alone, carboxy group-containing polymer (trade name: PEMULEN TR-2 Polymer, Lubrizol Advanced Materials, Inc.), and isostearic acid (trade name: Isostearic acid EX, Kokyu Alcohol Kogyo Co., Ltd.).

In particular, in Fig. 3, the following can be confirmed: amide I in the vicinity of 1530 cm⁻¹ (C=O stretching) and amide II in the vicinity of 1630 cm⁻¹ (N-H bending) shift to the higher wavenumber side, and meanwhile, C=O stretching of carboxylic acid of the carboxy group-containing polymer (trade name: PEMULEN TR-2 Polymer, Lubrizol Advanced Materials, Inc.) in the vicinity of 1700 cm⁻¹, and C=O stretching band of isostearic acid (trade name: Isostearic acid EX, Kokyu Alcohol Kogyo Co., Ltd.) shift to the higher wavenumber side. This is considered to indicate that the amide bond portion of the amide alcohol and the carboxy group of the carboxy group-containing polymer, as well as the amide bond portion of the amide alcohol and the carboxy group of the isostearic acid are respectively hydrogen bonded, thereby forming a complex.

Without being bound by any theory, it is believed that in both non-neutralized and neutralized states, nitrogen of the amide bond portion of the amide alcohol and the carboxy group of the carbomer form a hydrogen bond and exerts emulsifying ability.

Without being bound by any theory, it is considered as follows: in a non-neutralized state, the carboxy group of alkyl-modified carbomer and of isostearic acid becomes -COOH, forming a hydrogen bond with the carbonyl group of the amide bond of amide alcohol, and the complex is hydrophobic; when this complex is gradually neutralized, a part of the carboxy group is dissociated into COO⁻ to regain hydrophilicity, and the complex becomes to have a hydrophobic part and a hydrophilic part and exerts a higher emulsifying ability.

Incidentally, Example 2 comprising isostearic acid provided a more stable W/O emulsion as compared with Example 1 not comprising isostearic acid. The emulsified state is shown in Fig. 4.

It can be understood that even when isostearic acid is not used, a complex is formed with an amide alcohol and a carboxylic acid-containing polymer; however, by also using isostearic acid, a complex with higher hydrophilicity and higher W/O emulsifying ability is considered to be formed.

Emulsions of different formulations described in each table were prepared.

### Example 3 and comparative example 3

### [Table 3]

**Table 3: With or without addition of amide alcohol**

| | Ingredient name | Ingredient labeling name | Example 3 | Comparative example 3 |
|---|---|---|---|---|
| Aqueou s phase | Ion exchanged water | Water | 34.9 | 34.9 |
| | Glycerin | Glycerin | 5.0 | 5.0 |
| | 1,3-Butylene glycol | Butylene glycol | 3.0 | 3.0 |
| | Diol PD | Pentylene glycol | 2.0 | 2.0 |
| | | | | |
| | PEMULEN TR-2 | (Acrylates/alkyl acrylate (C10-30)) crosspolymer | 0.2 | 0.2 |
| | | | | |
| Oil phase | Squalane | Squalane | 26.5 | 31.75 |
| | Neolight 100P | Isodecyl neopentanoate | 10.0 | 10.0 |
| | | | | |
| | Amide alcohol OLH | To be determined | 5.25 | - |
| | Isostearic acid EX | Isostearic acid | 5.9 | 5.9 |
| | BENTONE GEL ISD V | Isododecane, disteardimonium hectorite, propylene carbonate | 3.5 | 3.5 |
| | Rheopearl KL2 | Dextrin palmitate | 2.0 | 2.0 |
| | Rheopearl WX | Dextrin (palmitate/hexyldeca noate) | 1.5 | 1.5 |
| | | | | |
| Neutral izing agent | Potassium hydroxide (10%) | Potassium hydroxide, water | 0.25 | 0.25 |
| Total | | | 100.00 | 100.00 |
| Emulsion particle size (µm) | | | ∼20 | ∼50 |
| Viscosity (mPa·s) | | | 258000 (next day) (No.64, 1.5 rpm) | Unmeasurabl e |
| Stability (45°C, 1 month) | | | ○ | - |

### (Production method and evaluation)

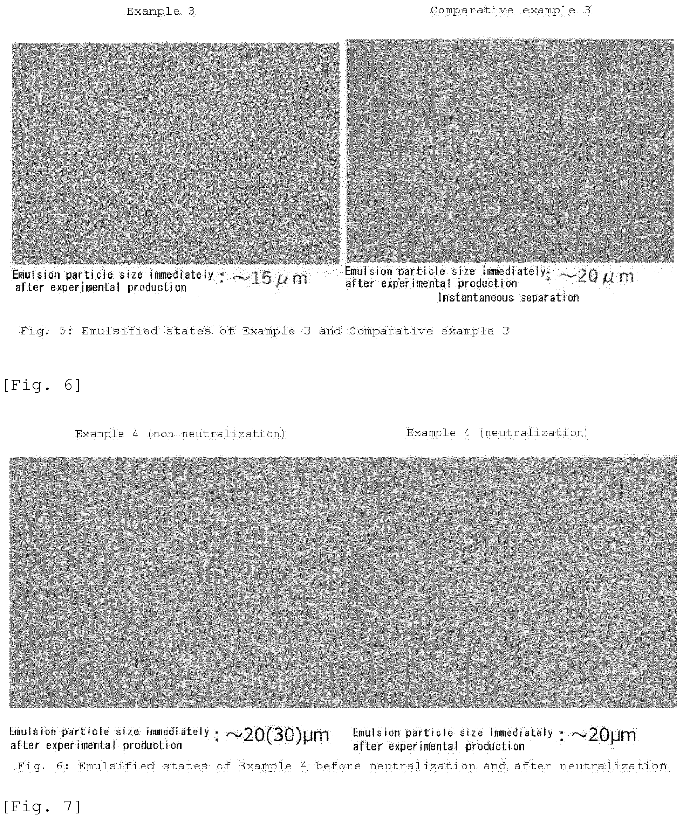

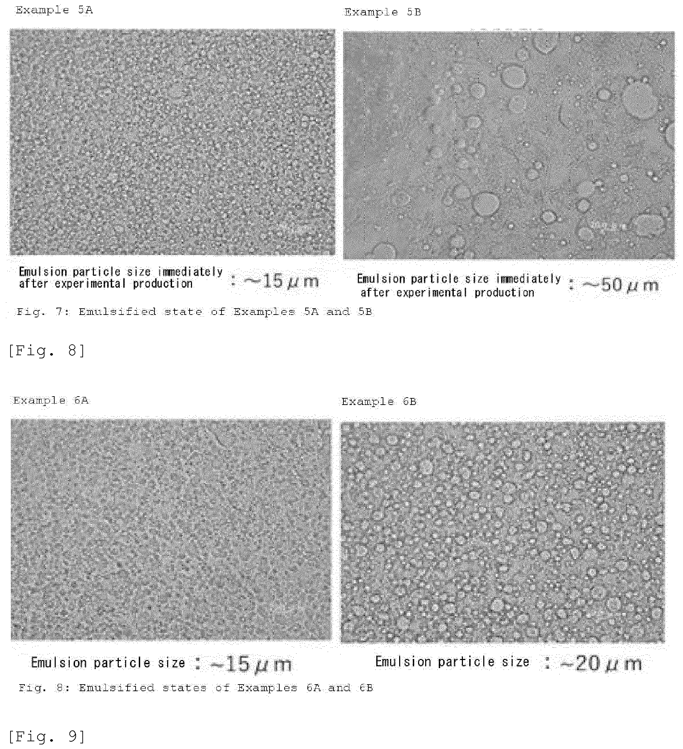

Example 3 and comparative formulation 3 were respectively prepared in the same manner as in Example 1, and the emulsion particle size and viscosity were measured. The emulsified state is shown in Fig. 5.

In Comparative example 3 lacking an amide alcohol, although emulsifying properties immediately after production were observed, separation advanced soon and an emulsion could not be obtained. On the other hand, in Example 3 comprising an amide alcohol, an emulsion that is stable for a long term was obtained.

### Example 4

### [Table 4]

**Table 4: Evaluation of addition of neutralizing agent**

| | Material name | Example 4 |
|---|---|---|
| Aqueous phase | Ion exchanged water | 34.9 |
| | Glycerin | 5.0 |
| | 1,3-Butylene glycol | 3.0 |
| | | |
| | PEMULEN TR-2 | 0.1 |
| | | |
| Oil phase | Squalane | 47.5 |
| | | |
| | Amide alcohol OLH | 4.0 |
| | BENTONE GEL ISD V | 5.0 |
| | | |
| Neutralizing agent | Potassium hydroxide (10%) | 0.5 |
| Total | | 100.0 |
| Emulsion particle size (µm) | | |
| Before addition of neutralizing agent | | ∼20 (30) |
| After addition of neutralizing agent | | ⇒∼20 |
| Viscosity (mPa·s) | | 223,000 (No.64, 1.5 rpm) |

### (Production method and evaluation)

Example 4 was prepared in the same manner as in Example 1, and the emulsion particle size and viscosity were measured. The emulsified states before neutralization and after neutralization are shown in Fig. 6.

Emulsifying properties were observed even before addition of a neutralizing agent, and more homogeneous emulsifying properties were obtained by adding a neutralizing agent.

### Examples 5A and 5B

### [Table 5]

**Table 5: Examination of the ratio of amide alcohol and carboxyl-containing polymer**

| | Material name | Example 5 A | Example 5 B |
|---|---|---|---|
| Aqueous phase | Ion exchanged water | 34.9 | 34.9 |
| | Glycerin | 5.0 | 5.0 |
| | 1,3-Butylene glycol | 3.0 | 3.0 |
| | Diol PD | 2.0 | 2.0 |
| | | | |
| | PEMULEN TR-2 | 0.2 | 0.05 |
| | | | |
| Oil phase | Squalane | 26.5 | 26.65 |
| | Neolight 100P | 10.0 | 10.0 |
| | | | |
| | Amide alcohol OLH | 5.25 | 5.25 |
| | Isostearic acid EX | 5.9 | 5.9 |
| | BENTONE GEL ISD V | 3.5 | 3.5 |
| | Rheopearl KL2 | 2.0 | 2.0 |
| | Rheopearl WX | 1.5 | 1.5 |
| | | | |
| Neutralizing agent | Potassium hydroxide (10%) | 0.25 | 0.25 |
| Total | | 100.00 | 100.00 |
| Ratio of amide alcohol/(carboxy group-containing polymer) | | 26.25 | 105 |
| Emulsion particle size (µm) | | ∼20 | ∼50 |
| Viscosity (mPa·s) | | 198,000 (next day) (No.64, 1.5 rpm) | 260,000 (next day) (No.64, 1.5 rpm) |

### (Production method and evaluation)

In the same manner as in Example 1, Example 5A and Example 5B in which blending ratios of amide alcohol and carboxy group-containing polymer were different were respectively prepared, and the emulsion particle size and viscosity were measured. The emulsified state is shown in Fig. 6.

In Example 4B having a high ratio of amide alcohol, an emulsion with larger emulsion particle size was obtained as compared with Example 4A. From this, it can be understood that the hydrophobicity becomes stronger by increasing the ratio of the amide alcohol, and the hydrophilicity becomes stronger as the proportion of the carboxy group-containing polymer becomes higher.

### Examples 6A and 6B

### [Table 6]

**Table 6: Addition of pentylene glycol**

| | Ingredient name | Example 6A | Example 6B |
|---|---|---|---|
| Aqueous phase | Ion exchanged water | 34.9 | 34.9 |
| | Glycerin | 5.0 | 5.0 |
| | 1,3-Butylene glycol | 3.0 | 3.0 |
| | Diol PD | 2.0 | - |
| | | | |
| | PEMULEN TR-2 | 0.2 | 0.2 |
| | | | |
| Oil phase | Squalane | 36.5 | 38.5 |
| | | | |
| | Amide alcohol OLH | 5.25 | 5.25 |
| | Isostearic acid EX | 5.9 | 5.9 |
| | BENTONE GEL ISD V | 3.5 | 3.5 |
| | Rheopearl KL2 | 2.0 | 2.0 |
| | Rheopearl WX | 1.5 | 1.5 |
| | | | |
| Neutralizing agent | Potassium hydroxide (10%) | 0.25 | 0.25 |
| Total | | 100.0 | 100.0 |
| Emulsion particle size (µm) | | ∼15 | ∼20 |
| Viscosity (mPa·s) | | 258,000 (next day) (No.64, 1.5 rpm) | 216,000 (next day) (No.64, 1.5 rpm) |

### (Production method and evaluation)

Example 6A (pentylene glycol blended) and Example 6B (without pentylene glycol) were respectively prepared in the same manner as in Example 1, and the emulsion particle size and viscosity were measured. The emulsified state is shown in Fig. 6.

It can be understood that by blending pentylene glycol, an emulsion showing better emulsifying properties can be obtained.

### Examples 7A, 7B and 7C

### [Table 7]

**Table 7: Formulation of different oil agents**

| | Material name | Example 7A | Example 7B | Example 7C |
|---|---|---|---|---|
| Aqueous phase | Ion exchanged water | 34.9 | 34.9 | 34.9 |
| | Glycerin | 5.0 | 5.0 | 5.0 |
| | 1,3-Butylene glycol | 3.0 | 3.0 | 3.0 |
| | Diol PD | 2.0 | 2.0 | 2.0 |
| | | | | |
| | PEMULEN TR-2 | 0.2 | 0.2 | 0.2 |
| | | | | |
| Oil phase | Squalane (IOB value : 0) | 36.5 | 25.5 | 20.5 |
| | Neolight 100P (IOB value : 0.22) | - | 10.0 | 20.0 |
| | | | | |
| | Amide alcohol OLH | 5.25 | 5.25 | 5.25 |
| | Isostearic acid EX | 5.9 | 5.9 | 5.9 |
| | BENTONE GEL ISD V | 3.5 | 3.5 | 3.5 |
| | Rheopearl KL2 | 2.0 | 3.0 | 3.0 |
| | Rheopearl WX | 1.5 | 1.5 | 1.5 |
| | | | | |
| Neutralizing agent | Potassium hydroxide (10% aqueous solution) | 0.25 | 0.25 | 0.25 |
| Total | | 100.0 | 100.0 | 105.0 |
| Ratio of nonpolar oil/(nonpolar oil/polar oil) | | 1.0 | 0.72 | 0.51 |
| Emulsion particle size (µm) | | ∼15 | ∼15 | ∼30 |
| Viscosity (mPa·s) | | 258,000 (next day) (No.64, 1.5 rpm) | 297,000 (that day) (No.64, 1.5 rpm) | 297,000 (that day) (No.64, 1.5 rpm) |

### (Production method and evaluation)

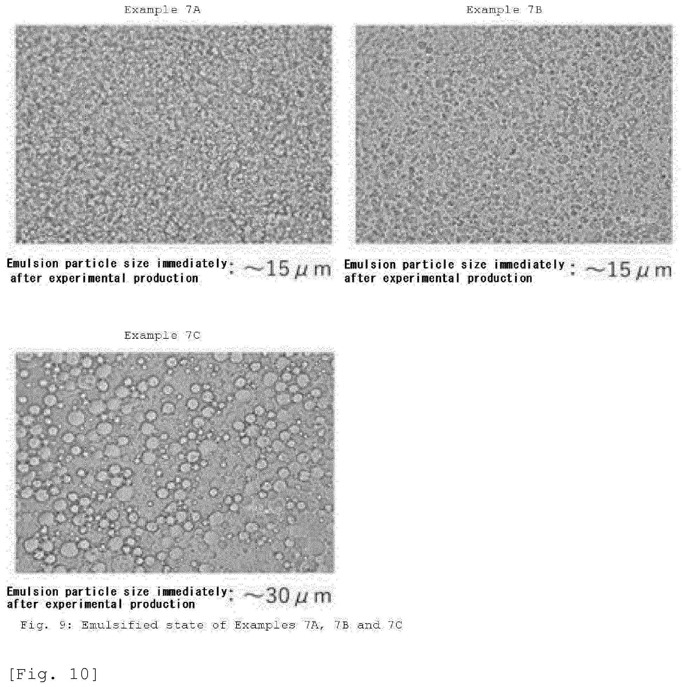

Example 7A (nonpolar oil only), Example 7B (nonpolar oil + polar oil), and Example 7C (nonpolar oil + polar oil) were respectively prepared in the same manner as in Example 1, and the emulsion particle size and viscosity were measured. The emulsified state is shown in Fig. 7.

It can be understood that as the ratio of the polar oil increases, the emulsion particle size increases, that is, as the proportion of the nonpolar oil increases, a more stable emulsion can be obtained.

In each of the above examples, the final emulsified composition was prepared by stirring and emulsifying the aqueous phase and the oil phase, then neutralizing by adding a neutralizing agent, and further stirring; here, differences in the emulsified states were examined between the following two processes: a process same as above in which a neutralizing agent is added after emulsification (hereinafter referred to as post-addition), and a process in which an aqueous phase containing a carboxyl-containing polymer is neutralized with an alkali, an oil phase is added thereto, then it is stirred and emulsified to prepare an emulsion (hereinafter referred to as pre-addition).

### Examples 8A and 8B

### [Table 8]

**Table 8: Timing of addition of neutralizing agent**

| | Material name | Example 8A (Neutralizing agent pre-addition) | Example 8B (Neutralizing agent post-addition) |
|---|---|---|---|
| Aqueous phase | Ion exchanged water | 34.9 | 34.9 |
| | Glycerin | 5.0 | 5.0 |
| | 1,3-Butylene glycol | 3.0 | 3.0 |
| | | | |
| | PEMULEN TR-2 | 0.2 | 0.2 |
| (Neutralizing agent) | | | |
| | Potassium hydroxide (10%) | 0.25 | - |
| Oil phase | Squalane | 38.5 | 38.5 |
| | | | |
| | Amide alcohol OLH | 5.25 | 5.25 |
| | Isostearic acid EX | 5.9 | 5.9 |
| | BENTONE GEL ISD V | 3.5 | 3.5 |
| | Rheopearl TL2 | 3.5 | 3.5 |
| | | | |
| Neutralizing agent | Potassium hydroxide (10%) | - | 0.25 |
| Total | | 100.0 | 100.0 |
| Emulsion particle size (µm) | | ∼20 | ∼20 |
| Viscosity (mPa·s) | | 121,000 (next day) (No.64, 1.5 rpm) | 165,000 (next day) (No.64, 1.5 rpm) |

The emulsion particle size and viscosity were measured in the same manner as in Example 1. The emulsified state is shown in Fig. 9.

Although slight differences were observed in the emulsified states due to the difference in the timing of addition of a neutralizing agent, it is understood that a favorable W/O emulsion can be prepared by any of the methods of pre-addition and post-addition.

Examples of W/O emulsion cosmetic compositions utilizing the complex of the present invention are shown below.

### [Example 9] Hair treatment cream

**[Table 9]**

| | | Blending amount (%) |
|---|---|---|
| (1) | Propylene glycol | 2.0 |
| (2) | Glycerin | 1.0 |
| (3) | Alkyl-modified carboxyvinyl polymer (Ingredient B) Trade name: Pemulen TR-1, Lubrizol Advanced Materials, Inc. | 0.1 |
| (4) | Amide alcohol OLH of structural formula (I-4) (Ingredient A) | 3.5 |
| | (Compound of structural formula (I) wherein R1 is C18, R2 is H, and R3 is C5) | |
| (5) | Squalane | 35.0 |
| | (Trade name: Olive squalene, Kokyu Alcohol Kogyo Co., Ltd.) | |
| (6) | Methyl phenyl polysiloxane | 1.0 |
| (7) | Organically modified clay mineral premix | 4.0 |
| | (Trade name: BENTONE IHD V (isohexadecane, disteardimonium hectorite, propylene carbonate), Elementis Specialties Inc.) | |
| (8) | Isostearic acid | 3.0 |
| | (Trade name: HAIMARIC MKH(R), Kokyu Alcohol Kogyo Co., Ltd.) | |
| (9) | Dextrin palmitate | 3.0 |
| | (Trade name: Rheopearl KL2, Rheopearl TL2, Chiba Flour Milling Co., Ltd.) | |
| (10) | Keratin hydrolysate | 1.0 |
| | Trade name: Promois WK-H, Seiwa Kasei Co., Ltd. | |
| (11) | Methylparaben | 0.1 |
| (12) | Octyl methoxycinnamate | 0.01 |
| (13) | Sodium hydroxide (10% aqueous solution) | 0.1 |
| (14) | Ion exchanged water | Balance |
| (15) | Perfume | 0.1 |

### <Production method>

(4) to (9) and (12) are uniformly mixed at 80°C (oil phase) . Meanwhile, (1) to (3), (10), (11) and (14) are uniformly mixed at 80°C (aqueous phase).

While adding the aqueous phase to the oil phase, the mixture is stirred with a disperser.

Next, (13) is added and emulsified. Upon completion of the emulsification, (15) was added and the emulsion was cooled to normal temperature to obtain a targeted viscosity of 220,000 mPa·s.

### [Example 10] W/O emulsion foundation

**[Table 10]**

| | | Blending amount (%) |
|---|---|---|
| (1) | Silicone-coated titanium oxide | 18.0 |
| (2) | Silicone-coated iron oxide (red) | 0.3 |
| (3) | Silicone-coated iron oxide (black) | 0.015 |
| (4) | Silicone-coated iron oxide (yellow) | 1.2 |
| (5) | Alkyl-modified carboxyvinyl polymer (Ingredient B) Trade name: Pemulen TR-2, Lubrizol Advanced Materials, Inc. | 0.2 |
| (6) | Decamethylcyclopentasiloxane | 35.0 |
| (7) | Trimethylsiloxysilicate/decamethylcyclopentasiloxane solution | 5.0 |
| | Trade name: X-21-5250, Shin-Etsu Chemical Co., Ltd. | |
| (8) | Amide alcohol LH of structural formula (I-3) (Ingredient A) | 3.0 |
| (9) | Sodium hydroxide | 0.1 |
| (10) | Ion exchanged water | Balance |
| (11) | Disteardimonium hectorite | 5.5 |
| | (Trade name: BENTONE 38V, Elementis Specialties Inc.) | |
| (12) | Isostearic acid | 5.0 |
| | (Trade name: Isostearic acid EX, Kokyu Alcohol Kogyo Co., Ltd.) | |
| (13) | Dextrin palmitate/hexyldecanoate | 3.5 |
| | (Trade name: Rheopearl WX, Chiba Flour Milling Co., Ltd. ) | |
| (14) | Perfume | Suitable amount |
| (15) | Pentylene glycol | 2.00 |
| | (Trade name: Diol PD-V, Kokyu Alcohol Kogyo Co., Ltd.) | |

### <Production method>

(1) to (4), (6) to (8), and (11) to (14) are uniformly dispersed at 80°C (oil phase).

Meanwhile, an aqueous phase of (5), (9) and (15) is uniformly dissolved and mixed at 80°C, and while gradually adding to the previously prepared oil phase, the mixture is stirred with a disperser.

Furthermore, a solution prepared with (9) and a part of (10) is added and emulsified. Upon completion of the emulsification, the emulsion is cooled to normal temperature to obtain a targeted W/O emulsion foundation having a viscosity of 450,000 mPa·s.

### [Example 11] Emollient cream

**[Table 11]**

| | | Blending amount (%) |
|---|---|---|
| (1) | Hydrogenated polyisobutene | 3.0 |
| (2) | Liquid paraffin | 3.0 |
| (3) | Isostearyl neopentanoate | 6.0 |
| | Trade name: Neolight 180P, Kokyu Alcohol Kogyo Co., Ltd. | |
| (4) | Decamethylcyclopentasiloxane | 5.0 |
| (5) | (Dimethicone/phenyl vinyl dimethicone) crosspolymer /diphenylsiloxy phenyl trimethicone mixture (Trade name: KSG-18A, Shin-Etsu Chemical Co., Ltd.) | 0.5 |
| (6) | Perfume | Suitable amount |
| (7) | Amide alcohol OLB of structural formula (I-1) (Ingredient A) | 6.0 |
| (8) | Ethylparaben | 0.1 |
| (9) | Butylparaben | 0.1 |
| (10) | Tocopherol | 0.5 |
| (11) | Alkyl-modified carboxyvinyl polymer (Ingredient B) | 0.15 |
| | (Trade name: Pemulen TR-2, Lubrizol Advanced Materials, Inc.) | |
| (12) | Carboxyvinyl polymer, (B) Ingredient | 0.15 |
| | (Trade name: Synthalen L, 3V Sigma USA Inc.) | |
| (13) | Polyethylene glycol 20000 | 1.0 |
| (14) | Crataegus cuneata fruit extract | 0.1 |
| (15) | Syzygium jambos leaf extract | 0.1 |
| (16) | Aloe extract | 0.1 |
| (17) | Sanguisorba officinalis root extract | 0.1 |
| (18) | Eugenia caryophyllus (clove) flower extract | 0.1 |
| (19) | Houttuynia cordata extract | 0.1 |
| (20) | Althaea officinalis root extract | 0.1 |
| (21) | Lithospermum officinale root extract | 0.1 |
| (22) | 1,3-Butylene glycol | 3.0 |
| (23) | Glycerin | 5.0 |
| (24) | Pentylene glycol | 2.0 |
| | (Trade name: Diol PD-V, Kokyu Alcohol Kogyo Co., Ltd.) | |
| (25) | Ion exchanged water | Balance |
| (26) | Potassium hydroxide | Suitable amount |
| (27) | Organically modified clay mineral premix | 4.0 |
| | (Trade name: BENTONE ISD V (isododecane, disteardimonium hectorite, propylene carbonate), Elementis Specialties Inc.) | |
| (28) | Dextrin palmitate/ethylhexanoate | 3.0 |
| | (Trade name: Rheopearl TT2, Chiba Flour Milling Co., Ltd.) | |

### <Production method>

(1) to (7), (27) and (28) are heated to 80°C and uniformly mixed (oil phase).

Meanwhile, (8) to (26) are uniformly mixed at 80°C (aqueous phase).

While gradually adding the aqueous phase to the oil phase at 80°C previously prepared, the mixture is stirred with a disperser.

Furthermore, a solution prepared with (26) and a part of (25) is added and emulsified. Upon completion of the emulsification, the emulsion is cooled to normal temperature to obtain a targeted W/O emulsion emollient cream having a viscosity of 280,000 mPa·s.

### [Example 12] Whitening cream

**[Table 12]**

| | | Blending amount (%) |
|---|---|---|
| (1) | Oleic acid | 3.5 |
| (2) | Isostearic acid (Trade name: Isostearic acid EX, Kokyu Alcohol Kogyo Co., Ltd.) | 0.5 |
| (3) | Squalane (Trade name: Olive squalene, Kokyu Alcohol Kogyo Co., Ltd.) | 25.0 |
| (4) | Triethylhexanoin (Trade name: TOG, Kokyu Alcohol Kogyo Co., Ltd.) | 3.0 |
| (5) | Hexyl laurate (Trade name: KAK HL, Kokyu Alcohol Kogyo Co., Ltd.) | 2.0 |
| (6) | Amide alcohol LH of structural formula (I-3) (Ingredient A) | 7.0 |
| (7) | Perfume | 0.1 |
| (8) | (Vinyl dimethicone/lauryl dimethicone) crosspolymer/isododecane mixture (Trade name: KSG-42, Shin-Etsu Chemical Co., Ltd,) | 0.5 |
| (9) | Tranexamic acid | 2.0 |
| (10) | Alkyl-modified carboxyvinyl polymer (Ingredient B) | 0.15 |
| | (Trade name: Pemulen TR-2, Lubrizol Advanced Materials, Inc.) | |
| (11) | Methylparaben | 0.1 |
| (12) | Phenoxyethanol | 0.1 |
| (13) | Dimethicone 6cs | 5.0 |
| (14) | Glycerin | 3.0 |
| | (Trade name: Triol VE, Kokyu Alcohol Kogyo Co., Ltd.) | |
| (15) | Pentylene glycol | 3.0 |
| | (Trade name: Diol PD-V, Kokyu Alcohol Kogyo Co., Ltd.) | |
| (16) | Hypericum perforatum extract | 0.1 |
| (17) | Leontopodium alpinum Extract | 0.1 |
| (18) | Royal jelly extract | 0.1 |
| (19) | Sodium ascorbyl phosphate | 0.1 |
| (20) | Ion exchanged water | Balance |
| (21) | Organically modified clay mineral premix | 3.5 |
| | (Trade name: BENTONE ISD V (isododecane, disteardimonium hectorite, propylene carbonate), Elementis Specialties Inc.) | |
| (22) | Dextrin myristate | 2.5 |
| | (Trade name: Rheopearl MKL, Chiba Flour Milling Co., Ltd.) | |

### <Production method>

(1) to (8), (21) and (22) are uniformly mixed at 80°C (oil phase).

Meanwhile, (9) to (20) are heated to 80°C and mixed uniformly (aqueous phase; since tranexamic acid has an action to increase pH, an extremely stable emulsion can be obtained without separately blending an alkali agent).

While gradually adding the aqueous phase to the oil phase at 80°C previously prepared, the mixture is stirred with a disperser.

Upon completion of the emulsification, the emulsion is cooled to normal temperature to obtain a targeted W/O emulsion whitening cream having a viscosity of 254,000 mPa·s.

### [Example 13] Whitening beauty essence

**[Table 13]**

| | | Blending amount (%) |
|---|---|---|
| (1) | Di(phytosteryl·2-octyldodecyl) N-lauroyl-L-glutamate | 0.5 |
| | (Trade name: Eldew PS-203, Ajinomoto Co., Inc.) | |
| (2) | Mineral oil | 5.0 |
| (3) | Squalane | |
| | (Trade name: Olive squalene, Kokyu Alcohol Kogyo Co., Ltd.) | 15.0 |
| (4) | Isododecane | 10.0 |
| (5) | Isodecyl neopentanoate | 3.0 |
| | (Trade name: Neolight 100P, Kokyu Alcohol Kogyo Co., Ltd.) | |
| (6) | Dimethicone 5cs | 1.0 |
| (7) | Amide alcohol LB of structural formula (I-2) (Ingredient A) | 5.5 |
| (8) | Alkyl-modified carboxyvinyl polymer (Ingredient B) | 0.1 |
| | (Trade name: Pemulen TR-1, Lubrizol Advanced Materials, Inc.) | |
| (9) | Carboxyvinyl polymer (Ingredient B) | 0.05 |
| | (Trade name: Carbopol ETD2050 Polymer, Lubrizol Advanced Materials, Inc.) | |
| (10) | Sodium hyaluronate | 0.1 |
| (11) | Glycerin | 5.0 |
| | (Trade name: Triol VE, Kokyu Alcohol Kogyo Co., Ltd.) | |
| (12) | 1,3-Butylene glycol | 3.0 |
| | (Trade name: Haisugarcane BG, Kokyu Alcohol Kogyo Co., Ltd.) | |
| (13) | Ethanol | 3.0 |
| (14) | 4-Isobutyl resorcinol | 0.25 |
| (15) | Ascorbic acid glucoside | 1.0 |
| (16) | Perfume | Suitable amount |
| (17) | Potassium hydroxide | Suitable amount |
| (18) | Sodium pyrosulfite | Suitable amount |
| (19) | Ion exchanged water | Balance |
| (20) | Pentylene glycol | 2.5 |
| | (Trade name: Diol PD-V, Kokyu Alcohol Kogyo Co., Ltd.) | |
| (21) | Organically modified clay mineral premix | 3.5 |
| | (Trade name: BENTONE ISD V (isododecane, disteardimonium hectorite, propylene carbonate), Elementis Specialties Inc.) | |
| (22) | Dextrin palmitate | 2.5 |
| | (Trade name: Rheopearl KL2, Chiba Flour Milling Co., Ltd.) | |
| (23) | Isostearic acid | 4.0 |
| | (Trade name: Isostearic acid EX, Kokyu Alcohol Kogyo Co., Ltd.) | |

### <Production method>

(1) to (7), (16), (21) to (23) are uniformly dissolved at 80°C (oil phase) .

Meanwhile, (8) to (21) are uniformly dissolved at 80°C (aqueous phase). The aqueous phase at 80°C is added to said oil phase at 80°C and stirred with a disperser.

To this, an aqueous solution in which (17) is dissolved in a part of (19) is added and emulsified by stirring with a disperser. Upon completion of the emulsification, a mixed solution of (13) and (10) is added and cooled to normal temperature to obtain a targeted whitening beauty essence having a viscosity of 157,000 mPa·s.

### [Example 14] W/O emulsion sunscreen

**[Table 14]**

| | | Blending amount (%) |
|---|---|---|
| (1) | Octyl p-methoxycinnamate | 3.0 |
| (2) | Glyceryl ethylhexanoate di-p-methoxycinnamate | 2.0 |
| (3) | 4-tert-butyl-4'-methoxydibenzoylmethane | 2.0 |
| (4) | Pentaerythrityl tetra(octanoate/p-methoxycinnamate) | 3.0 |
| (5) | Ethylhexyl isononanoate | 5.0 |
| | (Trade name: ES108109, Kokyu Alcohol Kogyo Co., Ltd.) | |
| (6) | Dimethicone 20cs | 3.0 |
| (7) | Squalane | 20.0 |
| | (Trade name: Olive squalene, Kokyu Alcohol Kogyo Co., Ltd.) | |
| (8) | Amide alcohol OLB of structural formula (I-1) (Ingredient A) | 2.2 |
| (9) | Glycerin | 4.0 |
| | (Trade name: Triol VE, Kokyu Alcohol Kogyo Co., Ltd.) | |
| (10) | Ion exchanged water | Balance |
| (11) | Dipropylene glycol | 1.0 |
| (12) | Methylparaben | 0.2 |
| (13) | Alkyl-modified carboxyvinyl polymer (Ingredient B) | 0.1 |
| | (Trade name: Pemulen TR-2, Lubrizol Advanced Materials, Inc.) | |
| (14) | Perfume | 0.1 |
| (15) | Triethanolamine | Suitable amount |
| (16) | Pentylene glycol | 2.5 |
| | (Trade name: Diol PD-V, Kokyu Alcohol Kogyo Co., Ltd. ) | |
| (17) | Organically modified clay mineral premix | 3.5 |
| | (Trade name: BENTONE ISD V (Isododecane, disteardimonium hectorite, propylene carbonate), Elementis Specialties Inc.) | |
| (18) | Dextrin palmitate | 2.5 |
| | (Trade name: Rheopearl KL2, Chiba Flour Milling Co., Ltd.) | |
| (19) | Isostearic acid | 4.0 |
| | (Trade name: Isostearic acid EX, Kokyu Alcohol Kogyo Co., Ltd.) | |

### <Production method>

(1) to (8), (14), (17) to (19) are uniformly dissolved at 80°C (oil phase) .

Meanwhile, (9) to (13) and (16) are uniformly mixed and dissolved at 80°C (aqueous phase).

This aqueous phase is gradually added to said oil phase heated to 80°C, and stirred with a disperser.

Furthermore, (15) is added and emulsified. Upon completion of the emulsification, the emulsion is cooled to normal temperature to obtain a targeted W/O emulsion sunscreen having a viscosity of 221,000 mPa·s.

The formulations of Examples 9-11, 13 and 14 can also be prepared by pre-adding a neutralizing agent such as potassium hydroxide, triethanolamine, sodium hydroxide, etc. to the aqueous phase.

### <O/W type emulsion>

### Example 15. Comparison of emulsifying properties with and without pentylene glycol

Emulsifying properties in oil-in-water type emulsified compositions using pentylene glycol were compared using the particle size.

**[Table 15]**

| | | Material name | Sample 1 | Sample 2 |
|---|---|---|---|---|
| | | | wt% | |
| Oil phase | 1 | Squalane | 12 | |
| | 2 | Hydrogenated rapeseed oil alcohol | 3.5 | |
| | 3 | Amide alcohol OLH | 2 | |
| Neutralizing agent | 4 | Potassium hydroxide (10%) | 1.5 | |
| Aqueous phase | **5** | Pentylene glycol | - | **5** |
| | 6 | Glycerin | 5 | |
| | 7 | 1,3-Butanediol | 3 | |
| | 8 | Carboxyvinyl polymer | 0.3 | |
| | 9 | Purified water | 72.7 | 67.7 |

Here, each raw material used in the Example is as follows: Squalane (trade name: Olive Squalane, Kokyu Alcohol Kogyo Co., Ltd.)
Hydrogenated rapeseed oil alcohol (trade name: Alcohol No. 20-B, Kokyu Alcohol Kogyo Co., Ltd.)
Amide alcohol OLH: Amide alcohol represented by formula (1-4) (Kokyu Alcohol Kogyo Co., Ltd.) 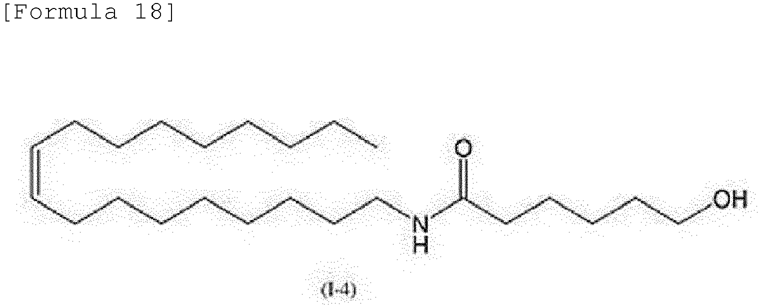 Pentylene glycol (trade name: Diol PD, Kokyu Alcohol Kogyo Co., Ltd.)
Glycerin (trade name: Triol VE, Kokyu Alcohol Kogyo Co., Ltd.)
1,3-Butanediol (trade name: Haisugarcane BG, Kokyu Alcohol Kogyo Co., Ltd.)
Carboxyvinyl polymer (trade name: Carbopol ETD2050 polymer, Lubrizol)

### Preparation method

(1) Each of oil phase and aqueous phase is uniformly dissolved at 80°C.
(2) While stirring the aqueous phase with Ajiter (an agitator), the oil phase is added (800 rpm).
(3) After emulsification, the mixture is stirred with Ajiter (2000 rpm, 3 min).
(4) Potassium hydroxide is added and further stirred (2000 rpm, 3 min).
(5) The mixture is slowly cooled to room temperature while stirring manually.

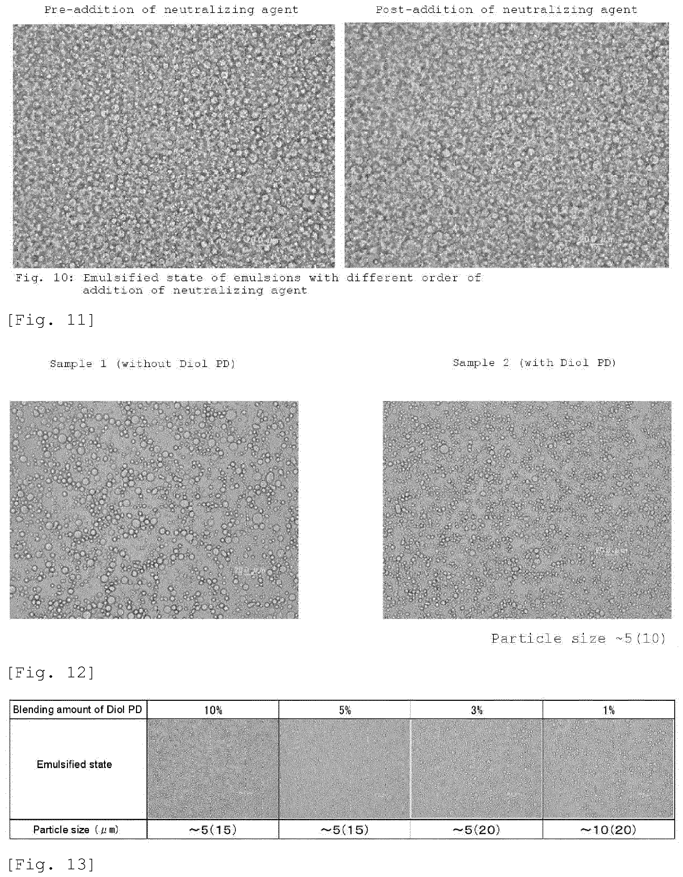

Results: The results are shown in Fig. 11. The emulsion containing Diol PD has a smaller particle size and improved emulsifying properties compared to the emulsion containing no Diol PD.

### Example 16. Comparison of emulsifying properties by content of pentylene glycol

The emulsifying properties in each oil-in-water emulsified composition were compared using the particle size while changing the content of pentylene glycol. In addition, as the preparation method, the method same as in Example 14 was used.

**[Table 16]**

| | | Material name | Sample 3 | Sample 4 | Sample 5 | Sample 6 |
|---|---|---|---|---|---|---|
| | | | wt% | | | |
| Oil, phase | 1 | Squalane | 12 | | | |
| | 2 | Hydrogenated rapeseed oil alcohol | 3.5 | | | |
| | 3 | Amide alcohol OLH | 2 | | | |
| Neutralizing agent | 4 | Potassium hydroxide (10%) | 1.5 | | | |
| Aqueous phase | **5** | **Pentylene glycol** | **10** | **5** | **3** | **1** |
| | 6 | Glycerin | 5 | | | |
| | 7 | 1,3-Butanediol | 3 | | | |
| | 8 | Carboxyvinyl polymer | 0.3 | | | |
| | 9 | Purified water | 62.7 | 67.7 | 69.7 | 71.7 |

Each raw material with the same name as Example 15 is the same product as what was described in Example 1. In addition, as mineral oil, trade name: Hicall K-230 (Kaneda Co., Ltd.) was used.

Results: The results are shown in Fig. 12. It was found that as the content of pentylene glycol increases, the particle size decreases, and in particular, the emulsification ratio improves by 5%.

From this result, it is likely that Diol PD interacts with amide alcohol.

### Example 17. Comparison of emulsifying properties by type of polyhydric alcohol

The emulsifying properties in each oil-in-water emulsified composition were compared using particle size while changing the type of polyhydric alcohol. Here, as the preparation method, the method same as in Example 14 was used.

**[Table 17]**

| | | Material name | wt% |
|---|---|---|---|
| Oil phase | 1 | Mineral oil | 12.0 |
| | 2 | Hydrogenated rapeseed oil alcohol | 3.5 |
| | 3 | Amide alcohol OLH | 2.0 |
| Aqueous phase | **4** | **Polyhydric alcohol** | **5.0** |
| | 5 | Glycerin | 5.0 |
| | 6 | 1,3-Butanediol | 3.0 |
| | 7 | 10% Potassium hydroxide solution | 3.0 |
| | 8 | Carboxyvinyl polymer | 0.3 |
| | 9 | Purified water | 72.7 |

Each raw material with the same name as Example 15 or 16 is the same product as what was described in Example 14 or 15.

Results: The results are shown in Fig. 13.

From this result, it was found that when pentylene glycol or 2-ethyl-1,3-hexanediol is used, the particle size becomes smaller and the emulsification ratio improves.

### Example 18. Example of improvement effect of emulsifying properties of higher fatty acid

**[Table 18]**

| | | Material name | Sample 7 | Sample 8 |
|---|---|---|---|---|
| | | | wt% | |
| Oil phase | 1 | Squalane | 12 | |
| | 2 | Hydrogenated rapeseed oil alcohol | 3.5 | |
| | 3 | Amide alcohol OLH | 2 | |
| | 4 | Isostearic acid | 2.0 | - |
| | | Trade name: Isostearic acid EX | | |
| | | from Kokyu Alcohol Kogyo Co., Ltd. | | |
| | 5 | Oleic acid | - | 2.0 |
| | | Trade name: Extra Olein from NOF Corporation | | |
| Neutralizing agent | 6 | Potassium hydroxide (10%) | 1.5 | |
| Aqueous phase | **7** | Pentylene glycol | **-** | **5** |
| | 8 | Glycerin | 5 | |
| | 9 | 1,3-Butanediol | 3 | |
| | 10 | Carboxyvinyl polymer | 0.3 | |
| | 11 | Purified water | 70.7 | 65.7 |
| Emulsion particle size (µm) | | | ∼5(20) | ∼5(10) |

### (Production method)

The production method is the same as the above-mentioned production method.

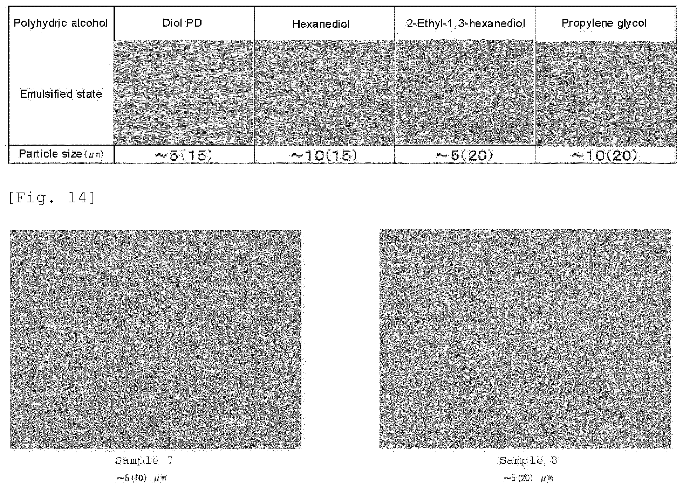

Results: The results are shown in Fig. 14.

### [Industrial applicability]

The present invention can provide a complex that enables preparation of emulsions, and an emulsion utilizing said complex, by combining an amide alcohol and a carboxy group-containing polymer.

Such an emulsion can be used in applications such as cosmetics.

## Claims

1. An emulsified composition, comprising an amide alcohol represented by formula (I): 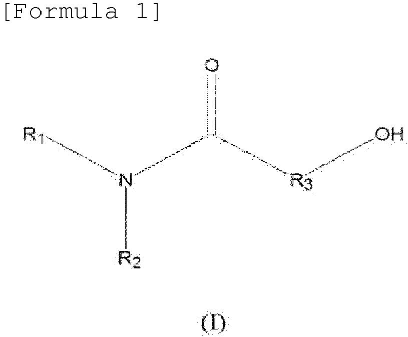 wherein
R₁ is a C6-C22 hydrocarbon group,
R₂ is H, or a C6-C22 hydrocarbon group,
R₃ is a linear or branched C2-C21 hydrocarbon group,
and a carboxy group-containing polymer.

2. The emulsified composition according to Claim 1, further comprising one or more polyhydric alcohols.

3. The emulsified composition according to Claim 2, wherein the polyhydric alcohol is pentylene glycol and/or 2-ethyl-1,3-hexanediol.

4. The emulsified composition according to any one of Claims 1 to 3, further comprising a higher fatty acid.

5. The emulsified composition according to any one of Claims 1 to 4, further comprising an oil gelling agent.

6. The emulsified composition according to any one of Claims 1 to 5, which is of W/O type.

7. The emulsified composition according to any one of Claims 1 to 6, which is of O/W type.

8. A complex formed by bonding an amide alcohol represented by formula (I): 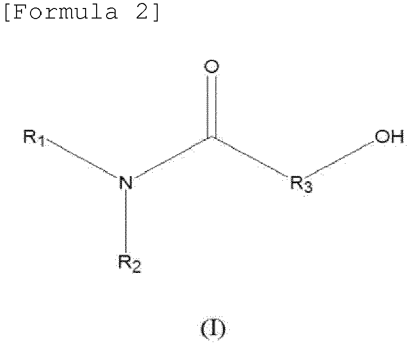 wherein
R₁ is a C6-C22 hydrocarbon group,
R₂ is H, or a C6-C22 hydrocarbon group,
R₃ is a linear or branched C2-C21 hydrocarbon group,
and a carboxy group-containing polymer,
and a higher fatty acid.

9. The complex according to Claim 8, further comprising one or more polyhydric alcohols.

10. A method for producing an emulsified composition according to Claim 1, wherein an oil phase comprising an amide alcohol represented by formula (I): 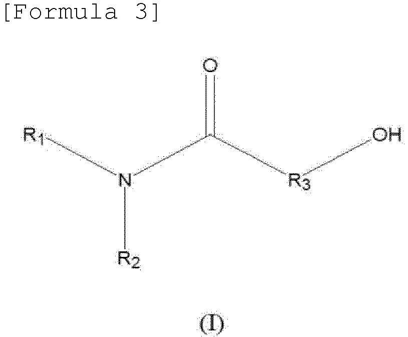 wherein
R₁ is a C6-C22 hydrocarbon group,
R₂ is H or a C6-C22 hydrocarbon group,
R₃ is a linear or branched C2-C21 hydrocarbon group,
and an aqueous phase comprising a carboxy-group containing polymer are mixed.

11. The method for producing an emulsified composition according to Claim 10, wherein the emulsified composition is of W/O type.

12. The method for producing an emulsified composition according to Claim 11, wherein the emulsified composition is of O/W type.
